# EUROPEAN PATENT SPECIFICATION

(11) **EP 3 790 888 B1**
(45) Date of publication and mention of the grant of the patent: **03.04.2024**
(21) Application number: 19723361.2
(22) Date of filing: 07.05.2019
(51) Int. Cl.: C07K 1/30, A23J 1/00, C07K 1/34, A23J 3/14, C07K 14/415

(54) **INTEGRATED PRECIPITATION AND MEMBRANE FILTRATION PROCESSES FOR ISOLATION OF POTATO PROTEINS**
INTEGRIERTES FÄLLUNGS- UND MEMBRANFILTRATIONSVERFAHREN ZUR ISOLIERUNG VON KARTOFFELPROTEINEN
PROCÉDÉS INTÉGRÉS DE PRÉCIPITATION ET DE FILTRATION MEMBRANAIRE POUR L'ISOLEMENT DE PROTÉINES DE POMME DE TERRE

(30) Priority: 07.05.2018 DK PA201870275
(43) Date of publication of application: 17.03.2021
(73) Proprietor: Duynie Holding B.V., 2407 AJ Alphen aan den Rijn (NL)
(72) Inventor: LIHME, Allan, Otto, Fog, 3520 Farum (DK); HANSEN, Marie, Bendix, 2000 Frederiksberg (DK); LINDVED, Bodil, Kjær, 3060 Espergærde (DK)
(74) Representative: Nederlandsch Octrooibureau
(86) International application number: PCT/EP2019/061686
(87) International publication number: WO 2019/215153

(56) References cited:
- WO-A1-97/03571
- WO-A1-2010/006621
- WO-A1-2012/116703
- WO-A1-2016/036243
- US-A1- 2010 048 873
- JAN BARTA ET AL: "EFFECT OF LOW-MOLECULAR ADDITIVES ON PRECIPITATION OF POTATO FRUIT JUICE PROTEINS UNDER DIFFERENT TEMPERATURE REGIMES", JOURNAL OF FOOD PROCESS ENGINEERING, vol. 31, no. 4, 9 July 2008 (2008-07-09), pages 533-547, XP55600973, US ISSN: 0145-8876, DOI: 10.1111/j.1745-4530.2007.00167.x
- KNUT OLAV STRÃ|TKVERN ET AL: "Recovery of Native Potato Protein Comparing Expanded Bed Adsorption and Ultrafiltration", FOOD AND BIOPROCESS TECHNOLOGY ; AN INTERNATIONAL JOURNAL, SPRINGER-VERLAG, NEW YORK, vol. 5, no. 5, 15 January 2011 (2011-01-15), pages 1939-1949, XP035067433, ISSN: 1935-5149, DOI: 10.1007/S11947-010-0494-2 cited in the application
- MICHAEL HOARE ET AL: "Precipitation of food proteins and their recovery by centrifuging and ultrafiltration", JOURNAL OF CHEMICAL TECHNOLOGY AND BIOTECHNOLOGY. BIOTECHNOLOGY, vol. 34, no. 3, 1 September 1984 (1984-09-01), pages 199-205, XP55538862, ISSN: 0264-3421, DOI: 10.1002/jctb.280340309 cited in the application
- N. DEVEREUX ET AL: "The Effect of Protein Precipitation on the Concentration of Proteins by Ultrafiltration", CHEMICAL ENGINEERING COMMUNICATIONS, vol. 45, no. 1-6, 16 July 1986 (1986-07-16), pages 255-276, XP055538864, US ISSN: 0098-6445, DOI: 10.1080/00986448608911389 cited in the application
- ZWIJNENBERG H J ET AL: "Native protein recovery from potato fruit juice by ultrafiltration", DESALINAT, ELSEVIER, AMSTERDAM, NL, vol. 144, no. 1-3, 10 September 2002 (2002-09-10), pages 331-334, XP004386240, ISSN: 0011-9164, DOI: 10.1016/S0011-9164(02)00338-7 cited in the application

## Description

### FIELD OF THE INVENTION

The present invention relates to integrated precipitation and membrane filtration methods for separation of one or more potato proteins from a group of impurities.

### BACKGROUND OF THE INVENTION

Techniques for industrial scale isolation of proteins from complex liquid raw materials have been a target of constant development for more than a century. Very many different methods based on various physico-chemical parameters have been described in the prior art but only few have found industrial applicability.

Purified proteins may be of value in widely different areas such as pharmaceutical, food, feed and technical applications and for each specific application there will be different target specifications for the purity and functionality of the protein. Likewise, the market value for a certain protein depends on the type of application. Thus, proteins intended for pharmaceutical applications have a much higher market value than proteins intended for food or feed applications. It is therefore crucial that any methodology chosen for the isolation of a protein, and its associated process cost, is carefully balanced against the value of the protein.

Membrane filtration is a widely and industrially used method for the isolation and concentration of proteins from complex mixtures. The fundamental separation principle is based on the passing of the liquid through semi-permeable membranes allowing only the passage of molecules smaller than the size of the porous structure of the membrane. Thus, membrane filtration separates molecules largely on the basis of their size and the availability of membranes with different pore sizes enables the separation of molecules and particles of varying size ranges. However, in order to achieve an efficient separation, the molecules to be separated must have very different sizes (such as at least 10 times different size). Molecules being closer in size will only be partially separated which may be detrimental to the product yield and thereby the economy of the separation process. Thus, membrane filtration, such as ultrafiltration and microfiltration, is intensively used in the dairy industry to separate proteins from milk and whey. Hereby highly purified protein mixtures devoid of lactose, milk fat and minerals may be produced at very large scale. Examples of such membrane-produced protein products are WPC 80 and WPI (Whey Protein Concentrate and Whey Protein Isolate respectively) which are mixtures of the dominant whey proteins e.g. beta-lactoglobulin, alpha-lactalbumin, immunoglobulins and lactoferrin with a very low content of lactose, minerals and lipids. On the other hand, and due to the comparable size of the proteins, membrane filtration is generally not industrially used for the separation of these protein mixtures into their single protein components.

The efficiency of membrane filtration is very sensitive to fouling of the membrane surface which generally leads to low flux rates, decreased permeability, reduced membrane service lifetime and increasing costs for cleaning of the membranes. Protein solutions to be treated by membrane filtration are therefore typically pretreated to remove insoluble and colloid particles and clarified to avoid such fouling issues. Protein solutions prepared by extraction from natural plant materials are generally very difficult to treat by membrane filtration under maintenance of commercially feasible flux rates and constant permeability.

Precipitation of proteins from aqueous solutions is widely used for large scale separation. Proteins may be precipitated by adding various agents such as organic solvents, lyotropic salts (such as ammonium sulfate) or polymers of different kind. Many food proteins are isolated from plant extracts (such as aqueous extracts of soy beans and peas) by so-called isoelectric precipitation which is based on the natural tendency of some proteins to become insoluble at pH values where the protein surface exhibits a near zero net charge. Isoelectric precipitation of proteins is generally a very low-cost operation. However, the method has limitations due to a rather low selectivity, co-precipitation of other unwanted substances and a narrow window of operation. A major drawback of the isoelectric precipitation method is that it is difficult to remove the co-precipitated impurities by washing of the precipitated proteins in a centrifuge because any change of the conditions (such as pH, temperature and ionic strength) may lead to solubilization and loss of the protein. Another major drawback of the isoelectric precipitation method is that only certain proteins will precipitate, leaving significant amounts of otherwise valuable proteins in the mother liquid and thereby lead to economic losses and environmental burdens from the associated waste water. Precipitation of proteins by the addition of chemical substances such as organic solvents, lyotropic salts and polymers is not generally applied for the industrial separation of food and feed grade proteins due to the high costs associated with the chemicals, the high costs of chemicals recycling and treatment of waste water and the need to completely remove these chemicals from the product after the precipitation process.

Precipitation of proteins from aqueous solutions may also be performed by the application of heat, such as heating to 60-100 degrees Celsius or 110-130 degrees Celsius under increased pressure, or even by heating combined with adjustment of pH to highly acidic pH values. Such processes are industrially applied, for example in order to precipitate potato proteins from potato fruit juice produced as a side-stream in the potato starch manufacturing industry. Such processes may be highly efficient; however, the proteins will be completely denatured by the process conditions. Typically, such treated proteins will be largely insoluble and any biological activity and functional characteristics will be lost.

Centrifugation is widely used for separation of precipitated proteins (curd) from aqueous solutions comprising impurities (whey). At industrial scale, for example for the isolation of soy protein isolates, decanter centrifuges are generally applied as the most efficient and cost-effective operation. The separated curd is typically washed and centrifuged again to remove beany flavors and other unwanted substances. The separation efficiency of such industrial scale centrifuges is very sensitive to alterations of the particle size distribution, particle density and stickiness of the curd as well as the liquid density and viscosity. Thus, it may prove difficult or even impossible to efficiently separate a precipitated protein in a large-scale application even though laboratory tests have shown good results. The sensitivity to these parameters further leads to significant limitations in the choice of operating conditions and the washing of the curd has to be performed within very narrow limits with respect to pH, salinity, temperature and composition of the washing solution. These limitations may in the end impart the quality of the final protein product due to the presence of e.g. off-flavors, pigments or even toxic compounds that have not been removed efficiently.

Compounds such as proteins and metabolites comprised in plants are valuable and useful in many different applications such as food and nutrition, medical treatments, cosmetics and acceptable process aids for industrial manufacture of the same. Particularly, such proteins and metabolites in significant crop plants, such as potatoes, are interesting and become even more valuable and useful in isolated form. Potatoes, for example, contain useful patatins and protease inhibitors which are desirable to use in isolated and more pure forms.

Kong et al. (Recovering proteins from potato juice by complexation with natural polyelectrolytes; International Journal of Food Science and Technology 2015, 50, 2160-2167) relates to characterization of potato proteins and their protein-polyelectrolyte complexes.

Waglay & Karboune (Potato Proteins: Functional Food Ingredients; Chapter 4; Advances in Potato Chemistry and Technology, 08 2016) disclose potato proteins prepared by various methods including thermal coagulation, acidic precipitation, precipitation with salt, ethanol, ammonium sulfate or CMC, anion-exchange chromatography or size exclusion separation. It is here disclosed that it is often undesirable to employ acidic precipitation due to protein denaturation, loss of functional characteristics and low yields.

Gerrit A Van Köningsveld (Journal of the Science of Food and Agriculture, vol 82, pp 134-142, 2001) disclose the solubility of potato proteins as influenced by pH and various additives.

Marie-Christine Ralet and Jacques GueHguen (LWT - Food Science and Technology Volume 33, Issue 5, August 2000, Pages 380-387) disclose pH dependent solubility, thermal coagulation and emulsifying properties of potato proteins.

Knut Olav Strætkvern & Jurgen G. Schwarz (Food and Bioprocess Technology, July 2012, Volume 5, Issue 5, pp 1939-1949) disclose membrane filtration and expanded bed adsorption for the isolation of native proteins from potato fruit water.

Michael Hoare and Peter Dunnill (J. Chem. Tech Biotechnol. Vol. 34B, 1984, pp 199-205) disclose the recovery of precipitated food proteins by centrifugation and ultrafiltration.

N. Devereux, M Hoare and P. Dunnill (Chem. Eng. Commun. Vol. 45, 1986, pp 255-276) disclose the effect of protein precipitation on the concentration of proteins by ultrafiltration.

Lotz el al (US 2010/0048873 A1) disclose a method for preparing coagulated plant proteins, including potato proteins. The preferred method for isolation of the coagulated protein is by decanters while membrane filtration is described as less advantageous due to higher time and effort consumption of this technology. The potato proteins are coagulated by a combination of acidic pH and high temperature treatment of potato juice which leads to protein denaturation and loss of water solubility and important functionalities of the protein.

However, many plants, including potatoes, also contain compounds that are undesirable or even poisonous in some applications. Particularly, potatoes (belonging to the nightshade family) contain several compounds which are undesired for some applications, while useful in other applications. Patatins and protease inhibitors are useful in nutrition and nutraceutical applications, while glycoalkaloids (toxic), lipoxygenase (rancidify fats/oils), polyphenol oxidase (oxidizes and tans food stuff) or phenolic compounds are not desired in nutrition and nutraceutical applications. On the other hand, isolated glycoalkaloids are useful in certain cosmetic or pharmaceutical applications. Accordingly, there is a need for methods for separating and/or isolating functional plant compounds to be used in industrial applications.

Isolation of highly purified proteins from plant extracts is a demanding task due to the extremely complex and reactive compositions achieved when the plant tissue is mechanically and/or chemically disrupted. Highly selective separation methods, such as adsorption chromatography, may relatively straightforward be applied to specifically adsorb and release the proteins free from contaminants but such methods have proven too costly in many applications targeting proteins for e.g. food applications. Other methods, like membrane filtration and classical separation of proteins by isoelectric precipitation or precipitation by the use of lyotropic salts (salting out) and organic solvents, are generally too unspecific when applied to crude plant extracts.

The increasing need for sustainable production of food and feed materials further emphasize the complexity involved in designing industrial scale processing methods that preserves the value of any given raw material and the product and side streams resulting from processing it. This means an increased need to avoid product losses and to avoid processing methods that destroy the value of the other components in the raw material such that they may be worked up as valuable products too and thereby increase the sustainability of the entire value chain. This is in contrast to many prior art processes that may focus mainly on one product to be produced from the raw material and where the potential value of side streams is neglected.

Especially in the field of potato protein isolation there has for many years been attempted many different techniques to economically produce food grade proteins out of the fruit juice released during the production of potato starches. It has, however, been difficult to achieve the quality needed for food proteins while at the same time applying an industrially applicable, robust and profitable processing scheme. For example, according to several scientific reports (e.g. Straetkvern KO & Schwarz JG (2012) Recovery of Native Potato Protein Comparing Expanded Bed Adsorption and Ultrafiltration. Food and Bioprocess Technology. 5(5), 1939-1949 and Zwijnenberg HJ, Kemperman AJB, Boerrigter ME, Lotz M, Dijksterhuis JF, Poulsen PE & Koops GH (2002) Native protein recovery from potato fruit juice by ultrafiltration. Desalination. 144(1-3), 331-334) ultrafiltration has low selectivity and only poorly separate polyphenols and brown polyphenol complexes from proteins thus giving a powder with a final brown hue and higher content of chlorogenic acids, and in addition it is often encountered with membrane concentration of potato fruit juice that fouling of the membranes lead to low flux rates, low system productivity and a shorter membrane lifetime. Zwijnenberg et al disclose a more than 80 % decrease of membrane permeability during concentration and diafiltration of potato fruit juice when testing several different types of ultrafiltration membranes.

Thus, the prior art generally points to significant disadvantages to the use of acidic precipitation of potato proteins due to protein denaturation and loss of functionality while membrane filtration is generally described as being inefficient due to membrane fouling, loss of productivity and poor separation selectivity and there is therefore a strong need to develop new and improved methods that solve these issues.

### SUMMARY OF THE INVENTION

The key findings of the present invention are that industrial scale separation of proteins from otherwise difficult to remove impurities may be efficiently achieved by certain integrated combinations of precipitation of the proteins followed by membrane filtration. The precipitated proteins are subjected to membrane filtration and diafiltration under conditions that allow the impurities to pass the membrane at high flux rates and without losses of product. The methods thus provided, and the particular order of the method steps, significantly improve what may be achieved by classical methods such as centrifugation.

Thus, in a first aspect the present invention relates to a method for separation of one or more proteins from a group of impurities in an aqueous solution, comprising:
a. providing an aqueous solution comprising the one or more proteins and a group of impurities
b. optionally subjecting the aqueous solution to a pretreatment to clarify and remove suspended non-soluble matter
c. precipitating the one or more proteins to create a suspension of precipitated protein in the solution containing impurities
d. subjecting the suspension to a membrane filtration process wherein at least one member of the group of impurities passes the membrane as a permeate and the one or more precipitated proteins are concentrated in the retentate
e. optionally diafiltering the retentate with one or more solvents to further remove impurities into the permeate
f. optionally re-solubilizing the precipitated one or more proteins
g. optionally separating the group of impurities in said permeate into at least two individual fractions

In particular, it has surprisingly been shown that a process combining precipitation of potato proteins at acidic pH values with membrane filtration of the suspended potato protein precipitate can be employed under conditions leading to high product purity while retaining both solubility and functionality as well as high operational membrane flux rates thus eliminating the disadvantages described in the prior art when employing these techniques separately.

Thus, a second aspect of the present invention relates to a method for separation of one or more potato proteins from a from a group of impurities in an aqueous solution, comprising:
a. providing an aqueous solution comprising the one or more potato proteins and a group of impurities
b. optionally subjecting the aqueous solution to a pretreatment to clarify and remove suspended non-soluble matter
c. precipitating the one or more potato proteins to create a suspension of precipitated potato protein in the solution containing impurities
d. subjecting the suspension to a membrane filtration process wherein at least one member of the group of impurities passes the membrane as a permeate and the one or more precipitated potato proteins are concentrated in the retentate
e. optionally diafiltering the retentate with one or more solvents to further remove impurities into the permeate
f. optionally re-solubilizing the precipitated one or more proteins
g. optionally separating the group of impurities in said permeate into at least two individual fractions

Other aspects of the technology are evident from the appended claims and the following description.

### LEGENDS TO THE FIGURES

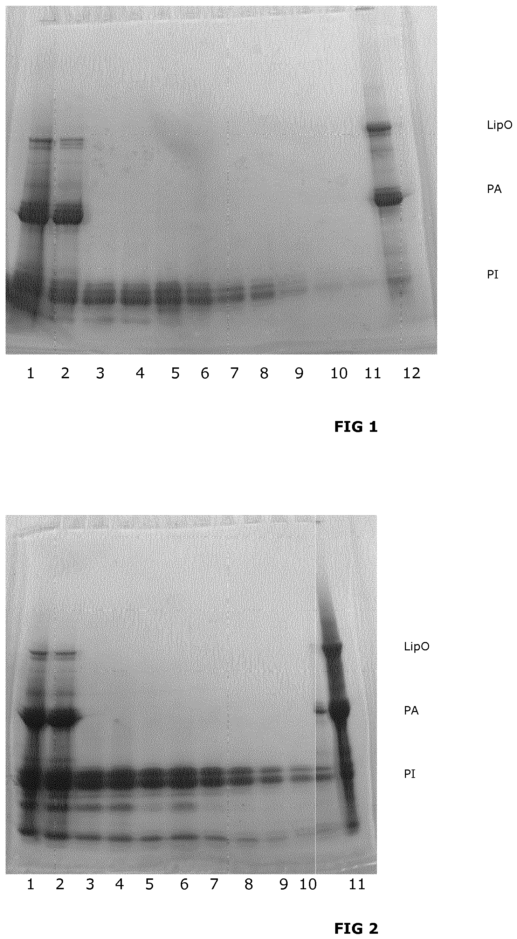

Figures 1-2 show SDS-PAGE analyses of the various solutions of the examples.

### DETAILED DESCRIPTION OF THE INVENTION

### Definitions:

The term "Impurities" means any substance being unwanted in the fraction of one or more proteins from which the impurities are to be separated. Thus, impurities may, for example, comprise other proteins, peptides, nucleotides, lipids, minerals, carbohydrates, phenols, glycoalkaloids, fatty acids, amines and glucosinolates.

The term "Group of impurities" means one or more impurities.

The term "Precipitating" means to transform a soluble protein into an insoluble (solidified) state for example in the form of large aggregates or complexes with other substances. The solidified compound is referred to as the "precipitate".

The term "Phenols" means a class of chemical compounds having one or more hydroxyl groups bonded directly to an aromatic hydrocarbon group. The simplest of the class is phenol, which is also called carbolic acid. Phenolic compounds are classified as simple phenols or polyphenols based on the number of phenol units in the molecule. Phenolic compounds are a large class of plant secondary metabolites, showing a diversity of structures, from rather simple structures, e.g. phenolic acids, through polyphenols such as flavonoids, that comprise several groups, to polymeric compounds, such as tannins.

The term "patatin", also denoted herein as "PA", means storage glycoproteins found in potatoes (Solanum tuberosum). Patatin represents a group of immunologically identical glycoprotein isoforms with molecular mass in the range of 40-43 kDa. Patatin also have phospolipase activity capable of cleaving fatty acids from membrane lipids. For purposes of the invention PA may be determined by different known assays, including SDS-PAGE combined with scanning densitometry as described herein (e.g. using a GS-900^{™} Calibrated Densitometer from BIO-RAD Laboratories, USA) including all protein bands in the molecular weight region between 35 kD and 60 kD in the PA category, ELISA testing using patatin specific antibodies, as well as enzymatic assays specific for the phospholipase activity (see e.g. Lipids, 2003, 38(6):677-82. "Determination of the phospholipase activity of patatin by a continuous spectrophotometric assay." Jiménez-Atiénzar M et al.)

The term "protease inhibitor", also denoted herein as "PI", means proteins, which possess molecular weights ranging from about 3 kD to about 35 kD, e.g. found in potatoes (Solanum tuberosum) and other plants such as soy and lupin, animals and microorganisms capable of inhibiting the activity of e.g. serine proteases, cysteine proteases, aspartate proteases, and metalloproteases. For purposes of the invention PI, in e.g. potato derived samples, may be determined by different known assays, including SDS-PAGE combined with scanning densitometry as described herein (e.g. using a GS-900^{™} Calibrated Densitometer from BIO-RAD Laboratories, USA) including all protein bands in the molecular weight region between 3 kD and 35 kD in the PI category, and more broadly by enzyme inhibition assays as generally described in the art (see e.g. The Open Food Science Journal, 2011, 5:42-46. "Quantitative Determination of Trypsin Inhibitory Activity in Complex Matrices". Robin E.J. Spelbrink et al.).

The term "polyphenol oxidase", also denoted herein as "PPO", means proteins found in nearly all plant tissues including potatoes (Solanum tuberosum), and can also be found in bacteria, animals, and fungi. Polyphenol oxidase (tyrosinase) (TY) is a bifunctional, copper-containing oxidase having both catecholase and cresolase activity. PPO causes the rapid polymerization of o-quinones to produce black, brown or red pigments (polyphenols) which cause fruit browning. The amino acid tyrosine contains a single phenolic ring that may be oxidised by the action of PPOs to form o-quinone. Hence, PPOs may also be referred to as tyrosinases.

The catalytic action of PPO has a negative impact on the quality of several fruit and vegetable crops and results in alteration of color, flavor, texture, and nutritional value. It is a limiting factor in the handling and technological processing of crops as peeled, sliced, bruised or diseased tissues rapidly undergo browning. For purposes of the invention PPO may be determined by different known assays as reviewed in: Journal of Food Biochemistry 2003, 27(5):361 - 422. "Physicochemical properties and function of plant polyphenol oxidase: A review". Ruhiye Yoruk et al.

The term "lipoxygenase", also denoted herein as "LipO", means proteins found in found in plants, animals and fungi capable of catalyzing the dioxygenation of polyunsaturated fatty acids. Lipoxygenases have food-related applications in bread making and aroma production but they also have negative implications for the color, off-flavour and antioxidant status of plant-based foods. In potatoes (Solanum tuberosum) lipoxygenase has a molecular weight of approx. 97 kD and can be detected by SDS-PAGE (see e.g. FEBS Journal, 2006, 273,3569-3584 "Patatins, Kunitz protease inhibitors and other major proteins in tuber of potato cv. Kuras" Guy Bauw et al.). For purposes of the invention LipO may be determined by different known assays, including SDS-PAGE combined with scanning densitometry as described herein (e.g. using a GS-900^{™} Calibrated Densitometer from BIO-RAD Laboratories, USA) as wells as enzyme activity assays as described in e.g. J. Agric. Food Chem., 2001, 49, 32-37. "Colorimetric Method for the Determination of Lipoxygenase Activity". Gordon E. Anthon et al.

The term "glycoalkaloid" or "alkaloid glucoside" means a family of chemical compounds derived from alkaloids in which sugar groups are appended. There are several that are potentially toxic, most notably those which are the poisons commonly found in the plant species Solanum dulcamara (nightshade). A prototypical glycoalkaloid is solanine (composed of the sugar solanose and the alkaloid solanidine), which is found in potatoes (Solanum tuberosum). For purposes of the invention glycoalkaloid may be determined by different known assays, including a standard HPLC assay as described Eng. Life Sci., 2005, 5, 562-567. "Optimization of glycoalkaloid analysis for us in industrial potato fruit juice downstreaming". Alt, V., Steinhof et al.

The term "legume" means a plant or its fruit or seed in the family Fabaceae (or Leguminosae). Legumes are grown agriculturally, primarily for their grain seeds called pulses.

The term "ligand" means a molecule comprising a functional group or moiety capable of binding to another molecule by non-covalent bonds, such as of hydrogen bonds, hydrophobic bonds, Π-Π (pi-pi) bonds and ionic bonds.

The term "anionic polymer" means a polymer that comprise one or more negatively charged moieties at a pH in the range of pH 3 to pH 13.

The term "dry weight" means the weight or mass of a substance remaining after removal of water by heating to constant weight at 110 degrees Celsius The dry weight per ml sample is thus the weight or mass of a substance remaining after removal of water by heating to constant weight at 110 degrees Celsius per ml sample applied to drying.

The term "isolating" or "separating" means any human intervention which change the relative amount of the compound compared to another selected constituent in a given matrix to a higher relative amount of the compound relative to the other constituent. In an embodiment, the compound may be isolated into a pure or substantially pure form. In this context, a substantially pure compound means that the compound preparation contains less than 10%, such as less than 8%, such as less than 6%, such as less than 5%, such as less than 4%, such as less than 3%, such as less than 2%, such as less than 1 %, such as less than 0.5% by weight of other selected constituents. In an embodiment, an isolated compound is at least 50% pure, such as at least 60% pure, such as at least 80% pure, such as at least 90% pure, such as at least 91% pure, such as at least 92% pure, such as at least 93% pure, such as at least 94% pure, such as at least 95% pure, such as at least 96% pure, such as at least 97% pure, such as at least 98% pure, such as at least 99% pure, such as at least 99.5% pure, such as 100 % pure by dry weight relative to other selected constituents.

The term "membrane separation process" refers to a process using a semi-permeable membrane, allowing only compounds having a size lower than a certain value to pass, to separate molecules of a higher size in a liquid continuous phase composition from molecules of a lower size. In this context, liquid continuous phase compositions are to be understood in the broadest sense, including both single phase compositions such as solutions and dual phase compositions such as slurries, suspensions or dispersions wherein a solid is distributed in a liquid phase.

The term "flux" or "flux rate" means the volume of liquid passing per hour through one square meter of membrane from the retentate side to the permeate side.

The term "permeability" means the concentration ratio for a specific substance on the membrane permeate side relative to the retentate side (C permeate/C retentate). Thus, the permeability is a measure for how efficient a substance is passing the membrane at any given point in time.

The term "retentate" means compounds which are not passing a selected membrane in a membrane separation process.

The term "permeate" or "filtrate" means compounds which pass a selected membrane in a membrane separation process.

The term "diafiltration" means a technique that uses membranes to completely remove, replace, or decrease the concentration of molecules of lower molecular weight than the compounds retained in the retentate. Such molecules typically comprise salts, solvents and other lower molecular weight compounds to be separated from solutions containing proteins and other high molecular weight biomolecules. The process selectively utilizes permeable (porous) membrane filters to separate the components of solutions and suspensions based on their molecular size. In a diafiltration process the retentate is added water or a solvent or buffer composition while the membrane filtration process continuously removes water, salts and low molecular weight compounds to the permeate side of the membrane.

The term "adsorption" means a process in which molecules from a liquid or dissolved solid adhere to a surface of a solid phase adsorbent. Likewise, an adsorbent (also named a solid phase adsorbent) is an insoluble, porous or non-porous material on which adsorption can occur.

The term "protein" means macromolecules consisting of one or more long chains of amino acid residues. In the context of this invention the term "protein" covers any chain length and thus includes small peptides and polypeptides.

The term "protein concentration" means the amount of protein per liter of a sample calculated as the total weight or mass of amino acids per liter as determined according to EUROPEAN PHARMACOPOEIA 5.0 section 2.2.56. AMINO ACID ANALYSIS or by determination of total nitrogen in a sample by the method of Kjeldahl using the conversion factor N x 6.25. All samples are dialyzed against demineralized water in dialysis tubing cellulose membrane (Sigma-Aldrich, USA, cat. No.: D9652) to remove any free amino acids and low molecular weight peptides prior to the amino acid determination.

The term "soluble" means solubility in water at a concentration of at least 0.1 g/L at 25 degrees Celsius.

The term "comprise" and "include" as used throughout the specification and the accompanying items/claims as well as variations such as "comprises", "comprising", "includes" and "including" are to be interpreted inclusively. These words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

Also in the following, the steps (a, a-1, a-2), (b, b-1, b-2), (c-1, c-2, c-3) etc. essentially refer to the same method step, with variations 1 and 2 as set out. Any general reference to a particular step, (e.g. step a), also refers to all variations of said steps (e.g. a-1, a-2). If a specific variation is meant, this will be referred to specifically (e.g. step a-1).

Many proteins precipitate spontaneously when the pH of the solution is near the isoelectric point of the protein and then solubilize again if the pH is adjusted to higher or lower pH values. The consequence of this behavior is that even though centrifugation may be the most cost effective method of separating the iso-electrically precipitated proteins from the bulk solution, centrifugation may not allow for an efficient washing of the precipitated proteins to further remove impurities with solvent conditions much different than the conditions employed to achieve the isoelectric precipitation because this may lead to full or partly product re-solubilization and unwanted losses.

It has now surprisingly been found that under certain conditions membrane filtration may be employed to efficiently separate one or more precipitated proteins from impurities even under conditions wherein the protein may be partly soluble. And even more surprisingly it has been found that membranes with a significantly higher permeability than what would be expected to retain the partly solubilized protein may be employed to achieve very high flux rates without significant loss of the protein to the permeate.

A method for separation of one or more proteins from a group of impurities in an aqueous solution is thus provided, the method comprising:
a. providing an aqueous solution comprising the one or more proteins and a group of impurities
b. optionally subjecting the aqueous solution to a pretreatment to clarify and remove suspended non-soluble matter
c. precipitating the one or more proteins to create a suspension of precipitated protein in the solution containing impurities
d. subjecting the suspension to a membrane filtration process wherein at least one member of the group of impurities passes the membrane as a permeate and the one or more precipitated proteins are concentrated in the retentate
e. optionally diafiltering the retentate with one or more solvents to further remove impurities into the permeate
f. optionally re-solubilizing the precipitated one or more proteins
g. optionally separating the group of impurities in said permeate into at least two individual fractions

In particular, it has surprisingly been shown that a process combining precipitation of potato proteins at acidic pH values with membrane filtration of the suspended potato protein precipitate can be employed under conditions leading to high product purity while retaining both solubility and functionality as well as high operational membrane flux rates thus eliminating the disadvantages described in the prior art when employing these techniques separately.

Thus, a second aspect of the present invention relates to a method for separation of one or more potato proteins from a from a group of impurities in an aqueous solution, comprising:
a. providing an aqueous solution comprising the one or more potato proteins and a group of impurities
b. optionally subjecting the aqueous solution to a pretreatment to clarify and remove suspended non-soluble matter
c. precipitating the one or more potato proteins to create a suspension of precipitated potato protein in the solution containing impurities
d. subjecting the suspension to a membrane filtration process wherein at least one member of the group of impurities passes the membrane as a permeate and the one or more precipitated potato proteins are concentrated in the retentate
e. optionally diafiltering the retentate with one or more solvents to further remove impurities into the permeate
f. optionally re-solubilizing the precipitated one or more proteins
g. optionally separating the group of impurities in said permeate into at least two individual fractions

Potato tuber proteins are highly nutritious and have many applications in human food systems, provided they are isolated with methods conserving their functionality and separating the proteins from toxic components and components that reduce palatability. The major soluble potato proteins are the protease inhibitor group of proteins (PI) and the patatin group of proteins (PA). As described in the literature PI is a group of proteins that are highly soluble in acidic, neutral and alkaline aqueous solutions while PA is soluble at alkaline pH but have a low solubility at about pH 4 and then again higher solubility at more acidic pH values. Thus, methods to isolate PA involving precipitation at slightly acidic pH at ambient temperature values followed by separation by centrifugation tend to give lower yields than other precipitation methods, for example precipitation at acidic pH values in combination with very high temperatures (e.g. in excess of 90 ⁰C). On the other hand, the combination of low pH and extreme temperatures result in complete denaturation of the protein and loss of functional properties (e.g. solubility, foaming, emulsification and gelling properties) in food applications.

However, in our search for improved methods for isolation of highly purified PA we have surprisingly found that even though the protein tends to be partly soluble at acidic pH values it may be efficiently retained by membranes having pore sizes even far exceeding the molecular weight of the PA molecules when performing a membrane filtration at acidic pH values wherein most of the PA is precipitated. It is hereby achievable to separate PA from PI as well as the lower molecular weight impurities including glycoalkaloids and phenols while retaining a high yield of PA and good functional properties as well as high flux rates and permeability in the membrane system.

In a preferred aspect of the invention the membrane filtration process of step d. is performed with a tangential cross-flow membrane and preferably a tubular polymeric membrane (e.g. a hollow fiber membrane) or a ceramic membrane.

In a preferred aspect of the invention the membrane filtration process of step d. is performed with a spinning disk membrane system.

In a preferred aspect of the invention the precipitation in step c. is performed at a pH in the range of 0.1 to 5.0, such as a pH in the range of 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.5 to 4.0, such as a pH in the range of 1.0 to 4.0, such as a pH in the range of 0.5 to 3.5, such as a pH in the range of 1.0 to 3.3.

In a preferred aspect of the invention the precipitation is performed at a temperature of 0.1-60 ⁰C, such as in the range of 2-25 ⁰C such as in the range of 20-60 ⁰C, such as in the range of 35-60 ⁰C, such as in the range of 40-55 ⁰C. In a preferred aspect the precipitation is performed in the range of 15-35 ⁰C, such as in the range of 18-30 ⁰C.

Further, we have surprisingly found that certain pigments, phenols and glycoalkaloids are more efficiently separated from the proteins by membrane filtration and/or diafiltration at very low pH values, such as for example below pH 3.5 such as below pH 2.5 and even below pH 1.8 without the loss of solubilized protein to the permeate.

Thus, in a preferred aspect of the invention the membrane filtration of step d. is performed at a pH in the range of pH 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.1 to 3.5, such as a pH in the range of 0.1 to 3.0, such as a pH in range of 0.1 to 2.5.

And further , in a preferred aspect of the invention the diafiltration of step e. is performed with an aqueous solution having a pH in the range of pH 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.1 to 3.5, such as a pH in the range of 0.1 to 3.0, such as a pH in range of 0.1 to 2.5.

Adjustment of pH may be performed with a broad range of food grade inorganic and organic acids, however, for the large-scale economy of the process the preferred acids may be selected from the group of sulfuric acid, hydrochloric acid, phosphoric acid, nitric acid, acetic acid, citric acid and lactic acid.

Precipitation of the proteins may further be achieved by complex formation with additives like polymers and silicates. However, for many applications this may an unnecessary additional cost to the process. Thus, in a preferred aspect of the invention no complexing additives are added to achieve or assist in the precipitation of protein in step c.

Thus, in a preferred aspect of the invention, the method comprises:
a-1. providing an aqueous solution comprising patatin, protease inhibitors, phenols and glycoalkaloids
b-1. optionally subjecting the aqueous solution to a pretreatment to clarify and remove suspended non-soluble matter
c-1. precipitating the patatin to create a suspension of precipitated patatin in the solution containing protease inhibitors and glycoalkaloids
d-1. subjecting the suspension to a membrane filtration process wherein the soluble protease inhibitors, phenols and glycoalkaloids pass the membrane as a permeate and the precipitated patatin is concentrated in the retentate
e-1. diafiltering the retentate with one or more solvents to further remove protease inhibitors, phenols and glycoalkaloids into the permeate
f-1. optionally re-solubilizing the precipitated patatin
g-1. separating the protease inhibitors and the glycoalkaloids into two individual fractions

In a preferred aspect the membrane filtration of step d. is performed with a membrane having a pore size in the range of 50.000 D to 1000.000 D, such as in the range of 100.000 D to 1000.000 D, such as in the range of 250.000 D to 1000.000 D. In step d-1, in particular, it is anticipated that the employed membrane has a porosity large enough to allow the protease inhibitors (they have a MW in the range of 5000 D to about 35000 D and may require a membrane of higher porosity to freely pass the membrane).

The porosity of membranes designed for microfiltration is generally indicated in nanometers or micrometers (microns). Thus, in a preferred aspect of the invention the membrane filtration process of step d-1. is performed with a microfiltration membrane with a pore size in the range of 0.05 micron to 5 micron, such as in the range of 0.05 micron to 3 micron, such as in the range of 0.05 micron to 2 micron, such as in the range of 0.1 to 1.8 micron, such as in the range of 0.2 micron to 2 micron, such as in the range of 0.5 to 1.7 micron.

In a preferred aspect of the invention the precipitation in step c-1. is performed at a pH in the range of 0.1 to 5.0, such as a pH in the range of 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.5 to 4.0, such as a pH in the range of 1.0 to 4.0, such as a pH in the range of 0.5 to 3.5, such as a pH in the range of 1.0 to 3.3.

Further, we have surprisingly found that certain pigments, phenols and glycoalkaloids are more efficiently separated from the PA by membrane filtration and/or diafiltration at very low pH values, such as for example below pH 3.5 such as below pH 2.5 and even below pH 1.8 without the loss of solubilized PA to the permeate.

Thus, in a preferred aspect of the invention the membrane filtration of step d-1. is performed at a pH in the range of pH 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.1 to 3.5, such as a pH in the range of 0.1 to 3.0, such as a pH in range of 0.1 to 2.5.

And further, in a preferred aspect of the invention the diafiltration of step e-1. is performed with an aqueous solution having a pH in the range of pH 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.1 to 3.0, such as a pH in range of 0.1 to 2.5.

For some technical and capital investment situations (e.g. if certain large-scale equipment is already available in the factory) it may be advantageous to combine the effect of centrifugation and membrane filtration in order to achieve the lowest cost process for manufacture. Also, it may be an advantage to combine the two techniques such that centrifugation of the suspension is performed prior to the membrane filtration under conditions where only a fraction of the precipitated patatin is removed. Under such conditions the centrifugation step may be performed at extremely high flow rates leading to significantly improved productivity relative to the investments in processing equipment.

Thus, in a preferred aspect the precipitated patatin of step c-1) is at least partly separated from the suspension prior to the membrane filtration in step d-1). In a preferred aspect 25-99 % of the precipitated patatin is separated, such as 50-99 % of the precipitated patatin is separated, such as 75-99 % of the precipitated patatin is separated, such as 85-99 % of the precipitated patatin is separated, such as 50-95 % of the precipitated patatin is separated, such as 75-95 % of the precipitated patatin is separated, such as 85-95 % of the precipitated patatin is separated, such as 50-90 % of the precipitated patatin is separated, such as 75-90 % of the precipitated patatin is separated, such as at least 75 % of the precipitated patatin is separated from the suspension prior to the membrane filtration step in step d-1).

In a preferred aspect the patatin is separated by centrifugation in a decanter.

In a preferred aspect the patatin is separated in a disk stack centrifuge.

Adjustment of pH may be performed with a broad range of food grade inorganic and organic acids, however, for the large-scale economy of the process the preferred acids may be selected from the group of sulfuric acid, hydrochloric acid, phosphoric acid, nitric acid, acetic acid, citric acid and lactic acid.

Precipitation of the proteins may further be achieved by complex formation with additives like polymers and silicates. However, for many applications this may an unnecessary additional cost to the process. Thus, in a preferred aspect of the invention no complexing additives are added to achieve or assist in the precipitation of protein in step c-1.

In a further preferred aspect of the invention PA and PI are simultaneously separated from lower molecular weight impurities such as pigments, glycoalkaloids and phenols. This may be efficiently achieved by performing the membrane filtration under conditions wherein the PA is precipitated, and the PI remain soluble, using a membrane having a porosity retaining both the precipitated PA and the soluble PI, but not the low molecular weight impurities.

Thus, in a further preferred aspect of the invention the method comprises:
a-2 providing an aqueous solution comprising patatin, protease inhibitors, phenols and glycoalkaloids
b-2 optionally subjecting the aqueous solution to a pretreatment to clarify and remove suspended non-soluble matter
c-2 precipitating the patatin to create a suspension of precipitated patatin in the solution containing protease inhibitors and glycoalkaloids
d-2 subjecting the suspension to a membrane filtration process wherein phenols and glycoalkaloids pass the membrane as a permeate and the precipitated patatin and the soluble protease inhibitors are concentrated in the retentate
e-2 diafiltering the retentate with one or more solvents to further remove phenols and glycoalkaloids into the permeate
f-2 optionally re-solubilizing the precipitated patatin
g-2 optionally separating the patatin and protease inhibitors into two individual fractions

Thus, in a preferred aspect the precipitated patatin of step c-2) is at least partly separated from the suspension prior to the membrane filtration in step d-2). In a preferred aspect 25-99 % of the precipitated patatin is separated, such as 50-99 % of the precipitated patatin is separated, such as 75-99 % of the precipitated patatin is separated, such as 85-99 % of the precipitated patatin is separated, such as 50-95 % of the precipitated patatin is separated, such as 75-95 % of the precipitated patatin is separated, such as 85-95 % of the precipitated patatin is separated, such as 50-90 % of the precipitated patatin is separated, such as 75-90 % of the precipitated patatin is separated, such as at least 75 % of the precipitated patatin is separated from the suspension prior to the membrane filtration step in step d-2).

In a preferred aspect the patatin is separated by centrifugation in a decanter.

In a preferred aspect the patatin is separated in a disk stack centrifuge.

Adjustment of pH may be performed with a broad range of food grade inorganic and organic acids, however, for the large-scale economy of the process the preferred acids may be selected from the group of sulfuric acid, hydrochloric acid, phosphoric acid, nitric acid, acetic acid, citric acid and lactic acid.

Precipitation of the proteins may further be achieved by complex formation with additives like polymers and silicates. However, for many applications this may an unnecessary additional cost to the process. Thus, in a preferred aspect of the invention no complexing additives are added to achieve or assist in the precipitation of protein in step c-2.

In a preferred aspect the membrane filtration of step d-2. is performed with a membrane having a pore size in the range of 1.000 D to 1.000.000 D, such as in the range of 5.000 D to 200.000 D, such as in the range of 7.000 D to 150.000 D, such as in the range of 8.000 D to 100.000 D, such as in the range of 5.000 D to 30.000 D.

In a preferred aspect of the invention the precipitation in step c-2. is performed at a pH in the range of 0.1 to 5.0, such as a pH in the range of 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.5 to 4.0, such as a pH in the range of 1.0 to 4.0, such as a pH in the range of 0.5 to 3.5, such as a pH in the range of 1.0 to 3.3.

Further, we have surprisingly found that certain pigments, phenols and glycoalkaloids are more efficiently separated from the PA by membrane filtration and/or diafiltration at very low pH values, such as for example below pH 3.5 such as below pH 2.5 and even below pH 1.8 without the loss of solubilized PA to the permeate.

Thus, in a preferred aspect of the invention the membrane filtration of step d-2. is performed at a pH in the range of pH 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.1 to 3.0, such as a pH in range of 0.1 to 2.5.

And further, in a preferred aspect of the invention the diafiltration of step e-2. is performed with an aqueous solution having a pH in the range of pH 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.1 to 3.5, such as a pH in the range of 0.1 to 3.0, such as a pH in range of 0.1 to 2.5.

Adjustment of pH may be performed with a broad range of food grade inorganic and organic acids, however, for the large-scale economy of the process the preferred acids may be selected from the group of sulfuric acid, hydrochloric acid, phosphoric acid, nitric acid, acetic acid, citric acid and lactic acid.

Precipitation of the proteins may further be achieved by complex formation with additives like polymers and silicates. However, for many applications this may an unnecessary additional cost to the process. Thus, in a preferred aspect of the invention no complexing additives are added to achieve or assist in the precipitation of protein in step c.

### Items

### Pretreatment

In an aspect of the invention the pretreatment of step b) is mandatory.

In an aspect of the invention the pretreatment comprise addition of one or more substances to flocculate the suspended non-soluble matter.

In an aspect of the invention the pretreatment comprises addition of calcium or magnesium ions to the solution.

In a preferred aspect of the invention the pretreatment comprises addition of phosphate and calcium ions to the solution.

In an aspect of the invention the pretreatment comprises addition of a soluble silicate to the solution.

In an aspect of the invention the pretreatment comprises addition of an anionic polymer to the solution.

In an aspect of the invention the pretreatment comprises addition of an anionic polymer selected from the group of anionic polysaccharides and synthetic anionic polymers.

In an aspect of the invention the pretreatment comprises addition of an anionic polymer selected from the group of alginate, carrageenan, carboxymethyl cellulose, carboxymethyl starch and pectins.

In a preferred aspect of the invention the pretreatment comprises heating the solution to a temperature in the range of 0.1-60 ⁰C, such as in the range of 5-25 ⁰C such as in the range of 30-60 ⁰C, such as in the range of 40-60 ⁰C, such as in the range of 45-55 ⁰C.

In a preferred aspect of the invention the pretreatment is performed at a pH in the range of pH 4.0 to pH 9.0, such as in the range of pH 4.5 to pH 6.5 such as in the range of pH 4.7 to pH 6.0, such as in the range of pH 4.7 to pH 5.8, such as in the range of pH 4.8 to pH 5.7.

In an aspect of the invention the pretreatment is performed at a pH in the range of pH 5.0 to pH 9.5 such as in the range of pH 5.2 to pH 9.0, such as in the range of pH 5.5 to pH 8.5, such as in the range of pH 5.5 to pH 8.0.

In a preferred aspect of the invention the pretreatment comprises centrifuging the solution at a g-force in the range of 1.500 g to 12.000 g, such as in the range of 1.500 g to 10.000 g, such as in the range of 1.500 g to 8.000 g, such as in the range of 2.000 g to 8.000 g, such as in the range of 2.000 g to 7.000 g, such as in the range of 2.500 g to 8.000 g, such as in the range of 2.500 g to 6.000 g, such as in the range of 3.000 g to 6.000 g.

In a preferred aspect of the invention the centrifugation is performed in a decanter centrifuge.

In an aspect of the invention the centrifugation is performed in a disk stack centrifuge.

In an aspect of the invention the centrifugation is performed in a nozzle centrifuge.

In an aspect of the invention the centrifugation is performed in two steps using first a decanter centrifuge and then a disk stack centrifuge or a nozzle centrifuge.

In a preferred aspect of the invention the pretreatment comprises a filtration step.

In a preferred aspect of the invention the pretreatment comprises a filtration step removing particles larger than 2 micron, such as larger than 5 micron, such as larger than 10 micron, such as larger than 15 micron, such as larger than 25 micron, such as larger than 50 micron, such as larger than 75 micron, such as larger than 100 micron, such as larger than 150 micron, such as larger than 200 micron, such as larger than 250 micron, such as larger than 300 micron.

In an aspect of the invention the pretreatment comprises a filtration step using a bag filter.

In an aspect of the invention the pretreatment comprises a filtration step using a filter press.

In an aspect of the invention the pretreatment comprises a filtration step using a vacuum filter.

### Precipitation

In a preferred aspect of the invention the precipitation in step c) comprise adjusting pH of the solution to be near the isoelectric point of the one or more proteins.

In a preferred aspect of the invention the precipitation in step c) comprise the addition of an anionic polymer.

In a preferred aspect of the invention the anionic polymer is a polysaccharide or a derivative hereof.

In an aspect of the invention the anionic polymer is an edible polymer.

In a preferred aspect of the invention the anionic polymer is chosen from the group of alginate, carrageenan, carboxymethyl cellulose, carboxymethyl starch, carboxymethyl dextran.

In an aspect of the invention the precipitation in step c) comprise the addition of a cationic polymer.

In an aspect of the invention the cationic polymer is chosen from the group of chitosan, polylysine, polyarginine.

In an aspect of the invention the precipitation in step c) comprise the addition of a soluble silicate, such as sodium silicate, sodium meta silicate.

In an aspect of the invention the precipitation in step c) comprise the addition of a metal ion selected from the group of calcium, magnesium, aluminium, iron, zinc or copper ions.

In an aspect of the invention the precipitation in step c) comprise the addition of a solid phase adsorbent to adsorb the one or more proteins.

In an aspect of the invention the solid phase adsorbent is an insoluble silicate compound such as clay or sand, glass, silica and derivatives hereof.

In an aspect of the invention the solid phase adsorbent is a porous polymer in the form of amorphous particles, beads or fibers.

In an aspect of the invention the solid phase adsorbent is a synthetic polymer.

In an aspect of the invention the solid phase adsorbent is a polysaccharide or derivatives hereof.

In an aspect of the invention the solid phase adsorbent comprises agarose, alginate, carrageenan, dextran, starch, cellulose or chitosan.

In an aspect of the invention the solid phase adsorbent comprises a covalently coupled ligand to adsorp the one or more proteins.

In an aspect of the invention the covalently coupled ligand comprise an aromatic ring system such as an aromatic acid, such as an aromatic amine, such as a phenol.

In an aspect of the invention the covalently coupled ligand comprise an ion exchange group such as a cationic group or an anionic group.

In a preferred aspect of the invention the precipitation is performed at a pH in the range of 0.1 to 9.5, such as a pH in the range of 0.1 to 8, such as a pH in the range of 0.1 to 7, such as a pH in the range of 0.1 to 6.5, such as a pH in the range of 0.1 to 6.0, such as a pH in the range of 0.1 to 5.5, such as a pH in the range of 0.1 to 5.0, such as a pH in the range of 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.5 to 4.0, such as a pH in the range of 1.0 to 4.0, such as a pH in the range of 0.5 to 3.5, such as a pH in the range of 1.0 to 3.3, such as a pH in the range of 3 to 8, such as a pH in the range of 3.5 to 8, such as a pH in the range of 4.0 to 8.0, such as a pH in the range of 4.5 to 8.0, such as a pH in the range of 5.0 to 8.0, such as a pH in the range of 5.5 to 8.0, such as a pH in the range of 5.5 to 7.5, such as a pH in the range of 5.5 to 7.0.

In an aspect of the invention the precipitation is performed at a temperature of 0.1-60 ⁰C, such as in the range of 2-25 ⁰C such as in the range of 20-60 ⁰C, such as in the range of 35-60 ⁰C, such as in the range of 40-55 ⁰C.

In an aspect of the invention the one or more proteins in step c) are at least 50 % precipitated, such as at least 60 % precipitated, such as at least 70 % precipitated, such as at least 80 % precipitated, such as at least 85 % precipitated, such as at least 90 % precipitated, such as at least 95 % precipitated.

### Membrane filtration

In a preferred aspect of the invention the membrane filtration process of step d) is a tangential flow filtration process.

In a preferred aspect of the invention the membrane filtration process of step d) is performed with a membrane with a pore size larger than 30.000 D, such as larger than 50.000 D such as larger than 100.000 D, such as larger than 200.000 D, such as larger than 300.000 D.

In a preferred aspect of the invention the membrane filtration process of step d) is performed with an ultrafiltration membrane having a pore size in the range of 1.000 D to 1.000.000 D, such as in the range of 5.000 D to 200.000 D, such as in the range of 7.000 D to 150.000 D, such as in the range of 8.000 D to 100.000 D, such as in the range of 5.000 D to 30.000 D, such as in the range of 30.000 D to 1000.000 D, such as in the range of 50.000 D to 1000.000 D, such as in the range of 100.000 D to 1000.000 D, such as in the range of 250.000 D to 1000.000 D.

In an aspect of the invention the membrane filtration process of step d) is performed with a polymeric membrane.

In a preferred aspect of the invention the membrane filtration process of step d. is performed with a ceramic membrane.

In a preferred aspect of the invention the membrane filtration process of step d. is performed with a ceramic membrane having channels of a diameter in the range of 2 mm to 10 mm.

In an aspect of the invention the membrane filtration process of step d) is performed with a spinning disk membrane.

In an aspect of the invention the membrane filtration process of step d) is performed with a spiral wound membrane.

In an aspect of the invention the membrane filtration process of step d) is performed with a hollow fiber membrane such as a hollow fiber membrane of the brand "Romicon" from Koch Membrane Systems (USA).

Preferably the Romicon membrane has a nominal pore size selected from the group of 500 kD, 100 kD, 50 kD, 30 kD and 10 kD.

Preferably the hollow fiber membrane has an inner diameter in the range of 1-5 mm, preferably in the range of 1-3 mm, preferably in the range of 1-2 mm.

In a preferred aspect of the invention the membrane filtration process of step d) is performed with a tubular membrane.

In a preferred aspect of the invention the membrane filtration process of step d) is performed with a microfiltration membrane with a pore size in the range of 0.05 micron to 5 micron, such as in the range of 0.05 micron to 3 micron, such as in the range of 0.05 micron to 2 micron, such as in the range of 0.1 to 1.8 micron, such as in the range of 0.2 micron to 2 micron, such as in the range of 0.5 to 1.7 micron.

In a preferred aspect the membrane filtration process of step d) is performed with a hydrophilic polymeric membrane such as a poly(ethersulfone) or polycarbonate membranes.

### Diafiltration

In a preferred aspect of the invention the diafiltration of step e) is mandatory.

In an aspect of the invention the diafiltration is performed with an aqueous solution having a pH in the range of pH of 0.1 to 9.5, such as a pH in the range of 0.1 to 8, such as a pH in the range of 0.1 to 7, such as a pH in the range of 0.1 to 6.5, such as a pH in the range of 0.1 to 6.0, such as a pH in the range of 0.1 to 5.5, such as a pH in the range of 0.1 to 5.0, such as a pH in the range of 0.1 to 4.5, such as a pH in the range of 0.5 to 4.5, such as a pH in the range of 0.8 to 4.5, such as a pH in the range of 0.9 to 4.5, such as a pH in the range of 0.5 to 4.0, such as a pH in the range of 1.0 to 4.0, such as a pH in the range of 0.5 to 3.5, such as a pH in the range of 1.0 to 3.3, such as a pH in the range of 3 to 8, such as a pH in the range of 3.5 to 8, such as a pH in the range of 4.0 to 8.0, such as a pH in the range of 4.5 to 8.0, such as a pH in the range of 5.0 to 8.0, such as a pH in the range of 5.5 to 8.0, such as a pH in the range of 5.5 to 7.5, such as a pH in the range of 5.5 to 7.0.

In a preferred aspect of the invention the diafiltration is performed with an aqueous solution having a pH in the range of pH 0.1 to 4.5, such as a pH in the range of 1.3 to 4.5, such as a pH in the range of 1.3 to 3.8, such as a pH in the range of 0.1 to 4.0, such as a pH in the range of 0.1 to 3.0, such as a pH in range of 0.1 to 2.5, such as a pH in the range of 0.1 to 2.0, such as a pH in the range of 0.2 to 1.5.

In a preferred embodiment the diafiltration step is performed in a separate membrane filtration unit.

In a further aspect the diafiltration solvent comprise a water miscible organic solvent such as ethanol, propanol, isopropanol and propylene glycol.

In an aspect of the invention the aqueous solution or the organic solvent used for diafiltration is treated in a regeneration process conditioning the solution or solvent for repeated use.

In a preferred aspect of the invention the regeneration process comprises a nanofiltration step.

In an aspect of the invention the regeneration process comprises an adsorption step for adsorption of low molecular compounds such as pigments, phenols and glycoalkaloids.

In an aspect of the invention the regeneration process comprises a distillation step.

### Proteins

### Potato proteins

In an aspect of the invention the one or more proteins is one or more potato proteins.

In an aspect of the invention the one or more proteins comprise patatin.

In an aspect of the invention the one or more proteins comprise lipoxygenase.

In an aspect of the invention the one or more proteins comprise polyphenol oxidase.

In an aspect of the invention the one or more proteins comprise patatin and the group of impurities comprise protease inhibitors and glycoalkaloids.

In an aspect of the invention the one or more proteins comprise patatin and protease inhibitors and the group of impurities comprise phenols and glycoalkaloids.

In a preferred aspect of the invention the protease inhibitors and the phenols and glycoalkaloids are separated by a further membrane filtration process.

In a preferred aspect of the invention the further membrane filtration process is an ultrafiltration process wherein the protease inhibitors are retained in the retentate and the phenols and glycoalkaloids are passing the membrane as a permeate.

In a preferred aspect of the invention the one or more proteins comprise patatin and the group of impurities comprise protease inhibitors, phenols and glycoalkaloids and the membrane filtration of step 4 is an ultrafiltration step wherein the phenols and glycoalkaloids pass the membrane as a permeate and the precipitated patatin and the soluble protease inhibitors are concentrated in the retentate.

### EXAMPLES

### Materials and methods

Chemicals used in the examples herein e.g. for preparing buffers and solutions are commercial products of at least reagent grade.

Water used for conducting the experiments is all de-ionized water.

Aqueous solutions comprising PA, PI, glycoalkaloids and phenols.

Potatoes of the variety Folva are obtained from a local supermarket.

The potatoes are washed and their surface are dried off before the potatoes are shredded with the peel while the liberated liquid (juice) concomitantly is separated from the main mass of insolubles using a commercial juicer (Nutrijuicer PRO) without diluting with water.

Sodium sulphite (10 wt%) is added immediately to the juice (10 ml sulphite per L juice). 10 kg of potatoes yields about 4.65 L of centrifuged juice (test solution 1) with a pH of 6.2 and a conductivity of 10.7 mS/cm, measured with a Seven2Go S3 conductivity meter from Mettler Toledo, Switzerland.

### Sodium alginate solution

Sodium alginate is obtained from Sigma Aldrich, USA (cat. no.: W201502). 15 g of sodium alginate is added up to 1 L of water and the alginate is solubilized by magnetic stirring yielding a 1.5 wt% sodium alginate solution.

### Buffer solutions

A 10 wt% sodium sulphite buffer solution is prepared by dissolving 10 g of sodium sulphite from Sigma Aldrich USA (cat. No.: 13471) in 100 mL water. pH was not adjusted. Measured to pH 7.7.
1 M NaOH
40 g of NaOH
Up to 1 L with water
10 % sulphuric acid solution
200 ml of 50 % sulphuric acid solution
Up to 1 L with water
0.1 M NaCl
5.84 g NaCl
Up to 1 L with water
SDS-PAGE electrophoresis reagents
   a) LDS sample buffer, 4X is obtained from Expedeon, USA (Cat.no.: NXB31010)
   b) SDS Run buffer, 20x is obtained from Expedeon, USA (Cat.no.: NXB50500)
   c) Precast 4-20% gradient gels are obtained from Expedeon, USA (Cat.no.: NXG42012K)
   d) Instant Blue Coomassie staining solution is obtained from Expedeon, USA (Cat.no.ISB1L).

### Assays

### a) SDS-PAGE electrophoresis

The samples produced in each example are analyzed using SDS-PAGE gel electrophoresis showing the protein composition in each sample. The SDS-PAGE gel electrophoresis is performed using an electrophoresis apparatus and precast 4-20% gradient gels from Expedeon USA (Cat.no.: NXG42012K). The protein samples are mixed with LDS sample buffer and incubated for 10 minutes at 70°C. The samples are applied to a precast gel and proteins are allowed run for one hour at 200 V 90 mA in the SDS Run buffer at non-reduced running conditions. The gel is developed in the staining solution for three hours and the protein bands are evaluated by visually inspection or analyzed by scanning densitometry to quantify the amount of specific proteins in the test solutions.

### b) Dry matter determination

A Sartorius moisture analyzer (MA37, Sartorius) is used to determine dry matter in a sample by applying 5-10 mL of a sample to the instrument. The sample is then dried at 110°C until constant weight and the remaining dry matter is determined and calculated by the instrument.

### c) Moisture determination

The moisture of a freeze dried sample was determined with the following method: 0.5 g of freeze dried sample was applied to the Sartorius moisture analyzer instrument (see above). The sample is then dried at 110°C until constant weight and the remaining dry matter is determined and calculated by the instrument. The moisture is calculated as: 100 % - the dry matter percentage.

### d) Determination of glycoalkaloids

A HPLC method for determination of glycoalkaloids is applied according to Alt, V., Steinhof, R., Lotz, M., Ulber, R., Kasper, C.,Scheper, T. Optimization of glycoalkaloid analysis for us in industrial potato fruit juice downstreaming. Eng. Life Sci. 2005, 5, 562-567.

Alpha-solanine (Sigma Aldrich, USA, cat no.: S3757) is used as a reference.

### e) Allkaline color test for phenolic compounds

A qualitative test for the content of complex phenolic compounds based on the development of color in alkaline medium. 0,5 ml sample is mixed with 3 ml 1 M sodium hydroxide and the absorbance at 360 nm is determined within one minute from mixing (BK-UV1800 spectrophotometer, Biobase, China). The result is calculated relative to the protein concentration in the sample as OD360 x 7/(mg protein per ml sample).

### f) Total true protein determination.

Standard amino acid quantification is performed according to EUROPEAN PHARMACOPOEIA 5.0 section 2.2.56. AMINO ACID ANALYSIS. All samples are initially dialyzed against demineralised water in dialysis tubing cellulose membrane (Sigma-Aldrich, USA, cat. No.: D9652) to remove any free amino acids and low molecular weight peptides.

### g) Protein determination.

The content of nitrogen in selected samples of final product was determined with elementary analysis. The protein content was calculated by multiplying the percentage of nitrogen with a factor of 6.25.

### Microfiltration and ultrafiltration

Solutions are membrane filtrated using a system from Spectrum Labs, USA, fitted with KrosFlo TFF system KMOi using hollow fiber membranes. A membrane with 0.65µm pore size and membrane area of 520 cm2 (Spectrum Labs, USA cat.no.: S02-E65U-07N), a membrane with 0.2 µm pore size and a membrane area of 470 cm2 (Spectrum Labs, USA cat.no.: S02-P20U-10N) and a membrane with 10.000 D pore size and a membrane area of 490 cm2 (Spectrum Labs, USA cat.no.: S02-E010-10N) are employed.

For the larger scale pilot runs a UNIVP system PAN 4010 from Koch Membrane System (USA) was employed. The system was used with hollow fiber units of the ROMICON type: 500 kD membrane, ROMICON HF UF Cartridge, 1018-1.0-43-PM500, PN 0720045; 30 kD membrane: ROMICON HF UF Cartridge, 1018-1.0-43-PM30, PN 0720039. The membrane area of both ROMICON membrane units was 0.09 m².

### Example 1. Isolating PA and PI in separate fractions from potato juice using alginate and microfiltration at pH 3.

3800 ml potato juice, test solution 1 (True protein content 10 g/L) is heated to 50°C and then centrifuged for three minutes at 1430 G, the supernatant is collected (test solution 2). 76 ml of 1.5 wt% alginate solution is added to test solution 2 and pH is adjusted to pH 3 with 10 % sulphuric acid. The solution is then incubated with mixing at 30°C for 5 minutes.

After incubation the solution is loaded onto a microfiltration unit with a 0.65 µm hollow fiber membrane. Cross flow is 2 L/min. During the initial concentration the permeate is collected in fractions of 500 ml (test solutions 3 through 9). When 250 ml retentate is remaining, pH in the retentate is adjusted to pH 0.9 with hydrochloric acid. 250 ml of water adjusted to pH 1.3 with hydrochloric acid is added to the retentate for washing of the retentate (diafiltration). 250 ml of permeate is then collected (test solution 10). Then another 250 ml of water pH 1.3 is added to the retentate and 250 ml of permeate is again collected (test solution 11). This procedure is performed three more times resulting in three additional permeate fractions (test solution 12, 13 and 14). Then 250 ml of water is added to the retentate and 250 ml of permeate is collected (test solution 15). This procedure is performed six more times resulting in 6 additional permeate fractions (test solution 16 through 21). Finally the pH in the retentate is adjusted to 8.5 and drained from the microfiltration unit (test solution 22) and freeze dried.

SDS-PAGE is performed on test solutions 1, 2, 3, 9, 10, 12, 14, 16, 18, 20, 21 and 22 as illustrated in figure 1.
Lane 1: Test solution 1, potato fruit juice
Lane 2: Test solution 2, potato fruit juice heated to 50°C and centrifuged, supernatant
Lane 3: Test solution 3, permeate fraction from 0.65 µm filter
Lane 4: Test solution 9, permeate fraction from 0.65 µm filter
Lane 5: Test solution 10, permeate fraction from 0.65 µm filter (water pH 1.3)
Lane 6: Test solution 12, permeate fraction from 0.65 µm filter (water pH 1.3)
Lane 7: Test solution 14, permeate fraction from 0.65 µm filter (water pH 1.3)
Lane 8: Test solution 16, permeate fraction from 0.65 µm filter (water, diafiltration)
Lane 9: Test solution 18, permeate fraction from 0.65 µm filter (water, diafiltration)
Lane 10: Test solution 20, permeate fraction from 0.65 µm filter (water, diafiltration)
Lane 11: Test solution 21, permeate fraction from 0.65 µm filter (water, diafiltration)
Lane 12: Test solution 22, retentate pH 8.5

### Results:

The average flux of the microfiltration process was 38 L/hr/m2 and no clogging of the hollow fibers was observed.

The SDS-PAGE of figure 1 illustrates that the permeate fractions contain only PI (see lane 3-11). These permeates can be pooled and ultrafiltrated and diafiltrated on a 10 kD membrane resulting in a pure PI product with a content of glycoalkaloids lower than 20 ppm.

The PA does not pass the membrane during the initial concentration, see lane 3 and 4 which show no PA bands in the permeate fractions. Surprisingly even when pH in the retentate is adjusted to pH 0.9, where PA normally partly dissolves, the PA does not pass the membrane during the retentate diafiltration wash with water/HCl at pH 1.3 (see lane 5-7) or the wash with water (diafiltration, see lane 8-11). It is only PI and the low molecular weight impurities that are recovered in these permeate fractions. The retentate contains a rather pure PA product with a very low concentration of PI (see lane 12).

The freeze-dried PA product is a white slightly yellowish powder.

Protein purity for PA: The nitrogen content of the freeze-dried sample was determined to be 11.99 %. Protein content = 11.99 %*6.25 = 74.94 % protein. The moisture is determined to be 7.49 %. This corresponds to a protein purity of 81 % on a dry matter basis.

The content of glycoalkaloids was determined to be lower than 20 ppm.

### Test for alkaline colored phenolic compounds:

A 1 % solution of the freeze dried PA product is made up in water (test solution 23). 3 ml 1 M NaOH is mixed with 0.5 ml test solution 23. To test the starting material 3 ml 1 M NaOH is mixed with 0.5 ml test solution 2 (potato juice supernatant after centrifugation).

Visual inspection of the color intensity of the dissolved PA (test solution 23) compared to the starting material (test solution 2) indicates that a large amount of colored substances (phenolic compounds) is present in the juice and very little is found in the re-dissolved PA solution (test solution 23). The absorbance of the samples is also measured at 360 nm. The result is calculated relative to the protein concentration in the sample as OD360 x 7/(mg protein per ml sample) see results in table 1.

**Table 1**

| Sample | OD 360 nm | OD360x7/mg protein per ml sample |
|---|---|---|
| PA product, solution 23 | 0.07 | 0.06 |
| Supernatant, potato juice, solution 2 | 1.051 | 0.74 |

Table 1 shows that the alkaline colored phenolic compounds is reduced with more than 90 % in the final product compared to the starting material.

Testing of the functional properties of the PA product showed that a 5 w/v % solution forms very stable emulsions with rapeseed oil and foams produced with a 4 w/v% solution gave an overrun of 850 % and a foam stability exceeding 10 hours.

### Example 2. Isolating PA and PI in separate fractions from potato juice using microfiltration at pH 2.7.

1600 ml potato juice, (test solution 1) is added calcium chloride to a final concentration of 20 mM and di-Sodium-hydrogen-phosphate to a final concentration of 10 mM, pH is adjusted to 7.5 with 1 M NaOH (test solution 2). Test solution 2 is incubated for 5 min, whereafter fibers and other insoluble material is removed by centrifugation (3 min at 1430 G). The supernatant is collected as test solution 3. Test solution 3 is adjusted to pH 2.7 with 10 % sulfuric acid. Test solution 3 is loaded onto a microfiltration unit with a 0.2 µm hollow fiber membrane. Cross flow is 1.2 L/min. During the initial concentration the permeate is collected in fractions of 466 ml (test solutions 4 through 6). When 200 ml retentate is remaining, 200 ml of 0.1 M NaCl is added to wash the retentate (dialfiltration). 200 ml of permeate is then collected (test solution 7). This procedure is performed four more times resulting in four additional permeate fractions (test solution 8, 9, 10 and 11). Then 200 ml of water is added to the retentate to wash (diafiltration) it further. Then 200 ml of permeate is collected (test solution 12). This procedure is performed four more times resulting in 4 additional permeate fractions (test solution 13 through 16). The pH in the retentate is adjusted to 9.2 and drained from the microfiltration unit (test solution 17) and freeze dried.

SDS-PAGE is performed on test solutions 1, 3, 4, 6, 8, 10, 11, 12, 13, 14 and 17 as illustrated in figure 2.
Lane 1: Test solution 1, potato fruit juice
Lane 2: Test solution 3, potato fruit juice treated with 20 mM CaCl2 and 10mM Na2HPO4 pH 7.5, supernatant after centrifugation
Lane 3: Test solution 4, permeate fraction from 0.2 µm filter
Lane 4: Test solution 6, permeate fraction from 0.2 µm filter
Lane 5: Test solution 8, permeate fraction from 0.2 µm filter (wash of retentate with 0.1 M NaCl)
Lane 6: Test solution 10, permeate fraction from 0.2 µm filter (wash of retentate with 0.1 M NaCl)
Lane 7: Test solution 11, permeate fraction from 0.2 µm filter (wash of retentate with 0.1 M NaCl)
Lane 8: Test solution 12, permeate fraction from 0.2 µm filter (Diafiltration of retentate with water)
Lane 9: Test solution 13, permeate fraction from 0.2 µm filter (Diafiltration of retentate with water)
Lane 10: Test solution 14, permeate fraction from 0.2 µm filter (Diafiltration of retentate with water)
Lane 11: Test solution 22, retentate pH 9.2, PA product

### Results:

The average flux of the microfiltration process was 32 L/hr/m2 and no clogging of the hollow fibers was observed.

The SDS-PAGE of figure 2 illustrates that all the permeate fractions contain only PI (see lane 3-10). These permeates can be pooled and ultrafiltrated and diafiltrated on a 10 kD membrane resulting in a pure PI product with a content of glycoalkaloids lower than 20 ppm. The PA does not pass the membrane during the initial concentration, see lane 3 and 4 which show no PA bands in the permeate fractions. The PA does not pass the membrane during the retentate wash with 0.1 M NaCl (see lane 5-7) or the wash with water (diafiltration, see lane 8-10). It is only PI that is recovered in these permeate fractions. The retentate contains a rather enriched PA fraction with a low concentration of PI (see lane 11).

Protein purity for PA: The nitrogen content of the freeze-dried sample was determined to be 12.62 %. Protein content = 12.62 %*6.25 = 78.9 % protein. The moisture content was determined to be 5.2 %. This corresponds to a protein purity of 83.2 % on a dry matter basis.

### Test for alkaline colored phenolic compounds:

A 1 % solution of the freeze-dried PA product is made up in water (test solution 23). 3 ml 1 M NaOH is mixed with 0.5 ml test solution 23. 3 ml 1 M NaOH is mixed with 0.5 ml test solution 2 (potato juice supernatant after centrifugation).

Visual inspection of the color intensity of the dissolved PA (test solution 23) compared to the starting material (test solution 2) indicates that a large amount of colored substances (phenolic compounds) is present in the juice and very little is found in the re-dissolved PA solution (test solution 23). The absorbance of the samples is also measured at 360 nm. The result is calculated relative to the protein concentration in the sample as OD360 x 7/(mg protein per ml sample) see results in table 2.

**Table 2**

| Sample | OD 360 nm | OD360x7/mg protein per ml sample |
|---|---|---|
| PA product, solution 23 | 0.20 | 0.17 |
| Supernatant, potato juice, solution 2 | 1.20 | 0.84 |

Table 2 shows that the alkaline colored phenolic compounds is reduced with about 80 % in the final product compared to the starting material.

The content of glycoalkaloids was determined to be lower than 20 ppm.

### Test for functionality

When whipped (4 w/v% dry matter solution) the PA product forms a stable foam with 850 % overrun that is stable for more than 2 hours.

### Example 3. Isolating PA and PI in one fraction from potato juice using ultrafiltration at pH 3.

1600 ml potato juice, test solution 1 (True protein content 10 g/L) is added calcium chloride to a final concentration of 20 mM and di-Sodium-hydrogen-phosphate to a final concentration of 10 mM, pH is adjusted to pH 6.8 with 1 M NaOH (test solution 2). Test solution 2 is incubated for 5 min, where after fibers and other insoluble material is removed by centrifugation (3 min at 1430 G). The supernatant is collected as test solution 3. Test solution 3 is adjusted to pH 2.9 with 10 % sulphuric acid and mixed at room temperature for 10 minutes. After incubation the solution is loaded onto an ultrafiltration unit with a 10 kD (10.000 D) hollow fiber membrane. Cross flow is 1.2 L/min. When 200 ml retentate is remaining, pH in the retentate is adjusted to pH 1.5 with sulfuric acid. 200 ml of water adjusted to pH 1.5 with sulfuric acid is added to the retentate for washing of the retentate (diafiltration). 200 ml of permeate is then collected (test solution 10). Then another 200 ml of water/sulfuric acid pH 1.5 is added to the retentate and 200 ml of permeate is again collected (test solution 11). This procedure is performed three more times resulting in three additional permeate fractions (test solution 12, 13 and 14). Then 200 ml of water is added to the retentate and 200 ml of permeate is collected (test solution 15). This procedure is performed six more times resulting in 6 additional permeate fractions (test solution 16 through 21). Finally, the pH in the retentate is adjusted to 8.5 and drained from the microfiltration unit (test solution 22) and freeze dried.

SDS-PAGE is performed on test solutions 1, 2, 3, 9, 10, 12, 14, 16, 18, 20, 21 and 22.

### Results:

The average flux of the microfiltration process was 23 L/hr/m2 and no clogging of the hollow fibers was observed.

The SDS-PAGE (not shown) illustrates that none of the permeate fractions contain visible protein bands. All proteins present in the starting material remain in the retentate.

Protein purity of freeze-dried retentate: The nitrogen content of the freeze-dried sample was determined to be 13.1 %. Protein content = 13.1 %*6.25 = 81.9 % protein. The moisture content was determined to be 6.2 %. This corresponds to a protein purity of 87.3 % on a dry matter basis.

### Test for alkaline colored phenolic compounds:

A 1 % solution of the freeze-dried PA/PI product is made up in water (test solution 23). 3 ml 1 M NaOH is mixed with 0.5 ml test solution 23. 3 ml 1 M NaOH is mixed with 0.5 ml test solution 2 (potato juice supernatant after centrifugation).

Visual inspection of the color intensity of the dissolved PA (test solution 23) compared to the starting material (test solution 2) indicates that a large amount of colored substances (phenolic compounds) is present in the juice and very little is found in the re-dissolved PA solution (test solution 23). The absorbance of the samples is also measured at 360 nm. The result is calculated relative to the protein concentration in the sample as OD360 x 7/(mg protein per ml sample) see results in table 2.

**Table 2**

| Sample | OD 360 nm | OD360x7/mg protein per ml sample |
|---|---|---|
| PA/PI product, solution 23 | 0.11 | 0.08 |
| Supernatant, potato juice, solution 2 | 1.20 | 0.84 |

Table 2 shows that the alkaline colored phenolic compounds is reduced with about 90 % in the final product compared to the starting material. The content of glycoalkaloids was determined to be lower than 20 ppm.

### Example 4. Pilot scale isolation of PA and PI in one fraction from potato juice using ultrafiltration at pH 3.5.

The procedure is performed at room temperature. 66 L potato juice, test solution 1 (True protein content 11 g/L) is added calcium chloride to a final concentration of 20 mM and di-Sodium-hydrogen-phosphate to a final concentration of 10 mM, pH is adjusted to pH 6.9 with 1 M NaOH (test solution 2). Test solution 2 is incubated for 5 min, where after fibers and other insoluble material is removed by centrifugation in a continuous Westfalia SA1 pilot plant separator. The light phase is collected as test solution 3. Test solution 3 is adjusted to pH 3.5 with 10 % sulphuric acid and mixed at room temperature for 10 minutes whereby the patatin precipitated and created a precipitate suspension in the potato fruit juice. After incubation the suspension is loaded onto an ultrafiltration unit (Koch Membrane Systems) mounted with a 30 kD ROMICON hollow fiber membrane. Cross flow is set at 15 L/min and the transmembrane pressure is maintained at approx. 1 bar by regulating feed and retentate valves. The system temperature throughout the run was maintained at 22-25 ⁰C by recirculating the retentate through the heat exchanger integrated in the Koch Membrane pilot system. The permeate was continuously collected into one permeate fraction.

When 55 L permeate was collected diafiltration was initiate by continuous addition of water to the remaining 11 L retentate, the water addition flow rate being adjusted to balance the permeate flow. Diafiltration was continued until a total of 205 L water had been added. Following diafiltration the retentate was added sodium hydroxide to reach a pH of 8,5 and the resulting solution was spray dried on a laboratory scale dryer (TP-S15, XIA'n Toption Instruments Co., Ltd, China).

The yield of off-white dried protein powder was 626 gram. The protein purity was 85 % and the total content of solanine (33 ppm) and chaconine (23 ppm) was 56 ppm.

The initial flux rate of the ROMICON membrane was found to be 33 LMH which remained largely constant throughout the test run.

### Example 5. Pilot scale isolation of PA and PI in two fractions from potato juice using combined centrifugation and ultrafiltration at pH 3.5.

The procedure is performed at room temperature. 62 L potato juice, test solution 1 (True protein content 11 g/L) is added calcium chloride to a final concentration of 20 mM and di-Sodium-hydrogen-phosphate to a final concentration of 10 mM, pH is adjusted to pH 6.9 with 1 M NaOH (test solution 2). Test solution 2 is incubated for 5 min, where after fibers and other insoluble material is removed by centrifugation in a continuous Westfalia SA1 pilot plant separator. The light phase is collected as test solution 3. Test solution 3 is adjusted to pH 3.5 with 10 % sulphuric acid and mixed at room temperature for 10 minutes whereby the patatin precipitated and created a precipitate suspension in the potato fruit juice. After incubation the suspension is centrifuged by passage through an MD80-Sn Laboratory Decanter (Lemitec, Germany) with the flow rate adjusted such that approx. 90 % of the precipitated patatin is retrieved in the heavy phase while leaving approx. 10 % of the precipitated patatin in the light phase. The light phase was hereafter loaded onto an ultrafiltration unit (Koch Membrane Systems) mounted with a 30 kD ROMICON hollow fiber membrane. Cross flow is set at 15 L/min and the transmembrane pressure is maintained at approx. 1.1 bar by regulating feed and retentate valves. The system temperature throughout the run was maintained at 22-25 ⁰C by recirculating the retentate through the heat exchanger integrated in the Koch Membrane pilot system. The permeate was continuously collected into one permeate fraction.

When 57 L permeate was collected diafiltration was initiate by continuous addition of water to the remaining 5 L retentate, the water addition flow rate being adjusted to balance the permeate flow. Diafiltration was continued until a total of 180 L water had been added. Following diafiltration the retentate was added sodium hydroxide to reach a pH of 8,5 and the resulting solution was spray dried on a laboratory scale dryer (TP-S15, XIA'n Toption Instruments Co., Ltd, China).

The yield of off-white dried protein powder was 280 gram. The protein purity was 88 % and the total content of solanine (31 ppm) and chaconine (27 ppm) was 58 ppm. When analyzed by SDS-PAGE the protein composition was highly enriched in the PI content with only a minor component being represented by PA

The initial flux rate of the ROMICON membrane was found to be 59 LMH which remained largely constant throughout the test run.

The heavy phase from the centrifugation of precipitated patatin was subsequently suspended in water and then also loaded onto the ultrafiltration unit (Koch Membrane Systems) now mounted with a 500 kD ROMICON hollow fiber membrane. Cross flow is set at 15 L/min and the transmembrane pressure is maintained at approx. 0.6 bar by regulating feed and retentate valves. The system temperature throughout the run was maintained at 22-25 ⁰C by recirculating the retentate through the heat exchanger integrated in the Koch Membrane pilot system. The permeate was continuously collected into one permeate fraction.

When the retentate was concentrated to a volume of 7 L diafiltration was initiated by continuous addition of water to the retentate, the water addition flow rate being adjusted to balance the permeate flow. Diafiltration was continued until a total of 110 L water had been added. Following diafiltration the retentate was added sodium hydroxide to reach a pH of 8,5 and the resulting solution was spray dried on a laboratory scale dryer (TP-S15, XIA'n Toption Instruments Co., Ltd, China).

The yield of off-white dried protein powder was 267 gram. The protein purity was 84 % and the total content of solanine (<20 ppm) and chaconine (<20 ppm) was less than 20 ppm. When analyzed by SDS-PAGE the protein composition was highly enriched in the PA content with only a trace component being represented by PI.

The initial flux rate of the ROMICON membrane was found to be as high as 180 LMH which slowly dropped to about 125 LMH during the test run.

## Claims

1. A method for separation of one or more potato proteins from a group of impurities in an aqueous solution, comprising;
a. providing an aqueous solution comprising the one or more potato proteins and a group of impurities
b. optionally subjecting the aqueous solution to a pretreatment to clarify and remove suspended non-soluble matter
c. precipitating the one or more potato proteins to create a suspension of precipitated potato protein in the solution containing impurities
d. subjecting the suspension to a membrane filtration process wherein at least one member of the group of impurities passes the membrane as a permeate and the one or more precipitated potato proteins are concentrated in the retentate
e. optionally diafiltering the retentate with one or more solvents to further remove impurities into the permeate
f. optionally re-solubilizing the precipitated one or more potato proteins
g. optionally separating the group of impurities in said permeate into at least two individual fractions

2. A method according to claim 1, comprising;
a-1. providing an aqueous solution comprising patatin, protease inhibitors, phenols and glycoalkaloids,
b-1.optionally subjecting the aqueous solution to a pretreatment to clarify and remove suspended non-soluble matter,
c-1. precipitating the patatin to create a suspension of precipitated patatin in the solution containing protease inhibitors and glycoalkaloids,
d-1.subjecting the suspension to a membrane filtration process wherein the soluble protease inhibitors, phenols and glycoalkaloids pass the membrane as a permeate and the precipitated patatin is concentrated in the retentate,
e-1.diafiltering the retentate with one or more solvents to further remove protease inhibitors, phenols and glycoalkaloids into the permeate,
f-1. optionally re-solubilizing the precipitated patatin,
g-1. separating the protease inhibitors and the glycoalkaloids into two individual fractions.

3. A method according to claim 1 comprising;
a-2 providing an aqueous solution comprising patatin, protease inhibitors, phenols and glycoalkaloids,
b-2 optionally subjecting the aqueous solution to a pretreatment to clarify and remove suspended non-soluble matter,
c-2 precipitating the patatin to create a suspension of precipitated patatin in the solution containing protease inhibitors and glycoalkaloids,
d-2 subjecting the suspension to a membrane filtration process wherein phenols and glycoalkaloids passes the membrane as a permeate and the precipitated patatin and the soluble protease inhibitors are concentrated in the retentate,
e-2 diafiltering the retentate with one or more solvents to further remove phenols and glycoalkaloids into the permeate,
f-2 optionally re-solubilizing the precipitated patatin,
g-2 optionally separating the patatin and protease inhibitors into two individual fractions.

4. The method according to anyone of claims 1-3 wherein said pretreatment step b) is mandatory.

5. The method according to anyone of claims 1-4 wherein said re-solubilization step f) is mandatory.

6. The method according to anyone of claims 1-5 wherein said separation step g) is mandatory.

7. The method according to anyone of the preceding claims wherein the pretreatment step b) comprises addition of one or more substances to flocculate the suspended non-soluble matter, preferablywherein the pretreatment step b) comprises addition of calcium or magnesium ions to the solution, more preferably wherein the pretreatment step b) comprises addition of phosphate ions and calcium or magnesium ions to the solution.

8. The method according to anyone of the preceding claims wherein the pretreatment step b) is performed at a pH in the range of pH 4.0 to pH 9.0, such as in the range of pH 4.5 to pH 6.5 such as in the range of pH 4.7 to pH 6.0, such as in the range of pH 4.7 to pH 5.8, such as in the range of pH 4.8 to pH 5.7.

9. The method according to anyone of the preceding claims wherein the precipitation in step c) is performed at a pH in the range of 0.1 to 5.0, such as a pH in the range of 0.1 to 4.5, such as a pH in the range of 0.5 to 4.5, such as a pH in the range of 0.8 to 4.5, such as a pH in the range of 0.9 to 4.5, such as a pH in the range of 1.0 to 4.5, such as a pH in the range of 1.3 to 4.5, such as a pH in the range of 1.3 to 3.8, such as a pH in the range of 0.1 to 4.0, such as a pH in the range of 0.5 to 4.0, such as a pH in the range of 1.0 to 4.0, such as a pH in the range of 0.5 to 3.5, such as a pH in the range of 1.0 to 3.3.

10. The method according to anyone of the preceding claims wherein the precipitation in step c) comprises the addition of an anionic polymer, preferably wherein the anionic polymer is an edible polymer chosen from the group of alginate, carrageenan, carboxymethyl cellulose, carboxymethyl starch, carboxymethyl and dextran.

11. The method according to anyone of the preceding claims wherein the membrane filtration process of step d) is a tangential flow filtration process wherein the membrane is a microfiltration membrane with a pore size in the range of 0.05 micron to 5 micron, such as in the range of 0.05 micron to 3 micron, such as in the range of 0.05 micron to 2 micron, such as in the range of 0.1 to 1.8 micron, such as in the range of 0.2 micron to 2 micron, such as in the range of 0.5 to 1.7 micron.

12. The method according to any of claims 1-10 wherein the membrane filtration process of step d) is a tangential flow filtration process performed with an ultrafiltration membrane having a pore size in the range of 1.000 D to 1.000.000 D, such as in the range of 5.000 D to 200.000 D, such as in the range of 7.000 D to 150.000 D, such as in the range of 8.000 D to 100.000 D, such as in the range of 5.000 D to 30.000 D, such as in the range of 30.000 D to 1000.000 D, such as in the range of 50.000 D to 1000.000 D, such as in the range of 100.000 D to 1000.000 D, such as in the range of 250.000 D to 1000.000 D.

13. The method according to anyone of the preceding claims wherein the membrane process of step d) is a tangential flow filtration process performed in a hollow fiber, tubular or spinning disk membrane system or wherein the membrane process of step d) is a tangential flow filtration process performed in a ceramic membrane system, or wherein the membrane process of step d) is a tangential flow filtration process performed in a spiral wound polymeric membrane system.

14. The method according to anyone of the preceding claims wherein the diafiltration of step e) comprises diafiltration at a pH in the range of 0.1 to 4.0, such as a pH in the range of 1.3 to 3.8, such as a pH in the range of 0.1 to 3.0, such as a pH in range of 0.1 to 2.5, such as a pH in the range of 0.1 to 2.0

15. The method according to anyone of the preceding steps wherein the membrane filtration of step d is performed at a pH in the range of 0.1 to 4.0, such as a pH in the range of 1.3 to 3.8, such as a pH in the range of 0.1 to 3.0, such as a pH in range of 0.1 to 2.5, such as a pH in the range of 0.1 to 2.0.

## Patentansprüche

1. Verfahren zur Trennung eines oder mehrerer Kartoffelproteine von einer Gruppe von Verunreinigungen in einer wässrigen Lösung, das Folgendes umfasst:
a. Bereitstellen einer wässrigen Lösung, die das eine oder die mehreren Kartoffelproteine und eine Gruppe von Verunreinigungen umfasst
b. optional Unterziehen der wässrigen Lösung einer Vorbehandlung zum Klären und Entfernen suspendierter nicht löslicher Stoffe
c. Ausfällen des einen oder der mehreren Kartoffelproteine, um eine Suspension von ausgefälltem Kartoffelprotein in der Verunreinigungen enthaltenden Lösung,
d. Unterziehen der Suspension einem Membranfiltrationsprozess, wobei mindestens ein Element der Gruppe von Verunreinigungen die Membran als Permeat passiert und das eine oder die mehreren ausgefällten Kartoffelproteine in dem Retentat konzentriert werden
e. optional Diafiltrieren des Retentats mit einem oder mehreren Lösungsmitteln, um Verunreinigungen in dem Permeat weiter zu entfernen
f. optional Wiederauflösen des ausgefällten einen oder der ausgefällten mehreren Kartoffelproteine
g. optional Trennen der Gruppe von Verunreinigungen in dem besagten Permeat in mindestens zwei einzelne Fraktionen

2. Verfahren nach Anspruch 1, das Folgendes umfasst:
a-1. Bereitstellen einer wässrigen Lösung, die Patatin, Proteaseinhibitoren, Phenole und Glykoalkaloide umfasst,
b-1. optional Unterziehen der wässrigen Lösung einer Vorbehandlung zum Klären und Entfernen suspendierter nicht löslicher Stoffe,
c-1. Ausfällen des Patatins, um eine Suspension von ausgefälltem Patatin in der Proteaseinhibitoren und Glykoalkaloide enthaltenden Lösung zu erzeugen,
d-1. Unterziehen der Suspension einem Membranfiltrationsprozess, wobei die löslichen Proteaseinhibitoren, Phenole und Glykoalkaloide die Membran als Permeat passieren und das ausgefällte Patatin in dem Retentat konzentriert wird,
e-1. Diafiltrieren des Retentats mit einem oder mehreren Lösungsmitteln, um die Proteaseinhibitoren, Phenole und Glykoalkaloide weiter aus dem Permeat zu entfernen,
f-1. optional Wiederauflösen des ausgefällten Patatins,
g-1. Trennen der Proteaseinhibitoren und der Glykoalkaloide in zwei einzelne Fraktionen.

3. Verfahren nach Anspruch 1, das Folgendes umfasst:
a-2 Bereitstellen einer wässrigen Lösung, die Patatin, Proteaseinhibitoren, Phenole und Glykoalkaloide umfasst,
b-2 optional Unterziehen der wässrigen Lösung einer Vorbehandlung zum Klären und Entfernen suspendierter nicht löslicher Stoffe,
c-2 Ausfällen des Patatins, um eine Suspension von ausgefälltem Patatin in der Proteaseinhibitoren und Glykoalkaloide enthaltenden Lösung zu erzeugen,
d-2 Unterziehen der Suspension einem Membranfiltrationsprozess, wobei Phenole und Glykoalkaloide die Membran als Permeat passieren und das ausgefällte Patatin und die löslichen Proteaseinhibitoren in dem Retentat konzentriert werden,
e-2 Diafiltrieren des Retentats mit einem oder mehreren Lösungsmitteln, um die Phenole und Glykoalkaloide weiter aus dem Permeat zu entfernen,
f-2 optional Wiederauflösen des ausgefällten Patatins,
g-2 optional Trennen des Patatins und der Proteaseinhibitoren in zwei einzelne Fraktionen.

4. Verfahren nach einem der Ansprüche 1-3, wobei der besagte Vorbehandlungsschritt b) obligatorisch ist.

5. Verfahren nach einem der Ansprüche 1-4, wobei der besagte Wiederauflösungsschritt f) obligatorisch ist.

6. Verfahren nach einem der Ansprüche 1-5, wobei der besagte Trennschritt g) obligatorisch ist.

7. Verfahren nach einem der vorhergehenden Ansprüche, wobei der Vorbehandlungsschritt b) die Zugabe einer oder mehrerer Substanzen zur Ausflockung der suspendierten nicht löslichen Stoffe umfasst, wobei vorzugsweise der Vorbehandlungsschritt b) die Zugabe von Kalzium- oder Magnesiumionen zu der Lösung umfasst, wobei besonders bevorzugt der Vorbehandlungsschritt b) die Zugabe von Phosphationen und Kalzium- oder Magnesiumionen zu der Lösung umfasst.

8. Verfahren nach einem der vorhergehenden Ansprüche, wobei der Vorbehandlungsschritt b) bei einem pH-Wert im Bereich von pH 4,0 bis pH 9,0 ausgeführt wird, wie zum Beispiel im Bereich von pH 4,5 bis pH 6,5, wie zum Beispiel im Bereich von pH 4,7 bis pH 6,0, wie zum Beispiel im Bereich von pH 4,7 bis pH 5,8, wie zum Beispiel im Bereich von pH 4,8 bis pH 5,7.

9. Verfahren nach einem der vorhergehenden Ansprüche, wobei die Fällung in Schritt c) bei einem pH-Wert im Bereich von 0,1 bis 5,0 ausgeführt wird, wie zum Beispiel einem pH-Wert im Bereich von 0,1 bis 4,5, wie zum Beispiel einem pH-Wert im Bereich von 0,5 bis 4,5, wie zum Beispiel einem pH-Wert im Bereich von 0,8 bis 4,5, wie zum Beispiel einem pH-Wert im Bereich von 0,9 bis 4,5, wie zum Beispiel einem pH-Wert im Bereich von 1,0 bis 4,5, wie zum Beispiel einem pH-Wert im Bereich von 1,3 bis 4,5, wie zum Beispiel einem pH-Wert im Bereich von 1,3 bis 3,8, wie zum Beispiel einem pH-Wert im Bereich von 0,1 bis 4,0, wie zum Beispiel einem pH-Wert im Bereich von 0,5 bis 4,0, wie zum Beispiel einem pH-Wert im Bereich von 1,0 bis 4,0, wie zum Beispiel einem pH-Wert im Bereich von 0,5 bis 3,5, wie zum Beispiel einem pH-Wert im Bereich von 1,0 bis 3,3.

10. Verfahren nach einem der vorhergehenden Ansprüche, wobei die Fällung in Schritt c) die Zugabe eines anionischen Polymers umfasst, wobei vorzugsweise das anionische Polymer ein essbares Polymer ist, das aus der Gruppe Alginat, Carrageenan, Carboxymethylzellulose, Carboxymethylstärke, Carboxymethyl und Dextran ausgewählt ist.

11. Verfahren nach einem der vorhergehenden Ansprüche, wobei der Membranfiltrationsprozess von Schritt d) ein Tangentialflussfiltrationsprozess ist, wobei die Membran eine Mikrofiltrationsmembran mit einer Porengröße im Bereich von 0,05 Mikron bis 5 Mikron ist, wie zum Beispiel im Bereich von 0,05 Mikron bis 3 Mikron, wie zum Beispiel im Bereich von 0,05 Mikron bis 2 Mikron, wie zum Beispiel im Bereich von 0,1 bis 1,8 Mikron, wie zum Beispiel im Bereich von 0,2 Mikron bis 2 Mikron, wie zum Beispiel im Bereich von 0,5 bis 1,7 Mikron.

12. Verfahren nach einem der Ansprüche 1-10, wobei der Membranfiltrationsprozess von Schritt d) ein Tangentialflussfiltrationsprozess ist, der mit einer Ultrafiltrationsmembran mit einer Porengröße im Bereich von 1.000 D zu 1.000.000 D ausgeführt wird, wie zum Beispiel im Bereich von 5.000 D bis 200.000 D, wie zum Beispiel im Bereich von 7.000 D bis 150.000 D, wie zum Beispiel im Bereich von 8.000 D bis 100.000 D, wie zum Beispiel im Bereich von 5.000 D bis 30.000 D, wie zum Beispiel im Bereich von 30.000 D bis 1000.000 D, wie zum Beispiel im Bereich von 50.000 D bis 1000.000 D, wie zum Beispiel im Bereich von 100.000 D bis 1000.000 D, wie zum Beispiel im Bereich von 250.000 D bis 1000.000 D.

13. Verfahren nach einem der vorhergehenden Ansprüche, wobei der Membranprozess von Schritt d) ein Tangentialflussfiltrationsprozess ist, der in einem Hohlfaser-, Rohr- oder Spinnscheibenmembransystem ausgeführt wird, oder wobei der Membranprozess von Schritt d) ein Tangentialflussfiltrationsprozess ist, der in einem Keramikmembransystem ausgeführt wird, oder wobei der Membranprozess von Schritt d) ein Tangentialflussfiltrationsprozess ist, der in einem spiralförmig gewickelten Polymermembransystem ausgeführt wird.

14. Verfahren nach einem der vorhergehenden Ansprüche, wobei die Diafiltration von Schritt e) Diafiltration bei einem pH-Wert im Bereich von 0,1 bis 4,0 umfasst, wie zum Beispiel einem pH-Wert im Bereich von 1,3 bis 3,8, wie zum Beispiel einem pH-Wert im Bereich von 0,1 bis 3,0, wie zum Beispiel einem pH-Wert im Bereich von 0,1 bis 2,5, wie zum Beispiel einem pH-Wert im Bereich von 0,1 bis 2,0.

15. Verfahren nach einem der vorhergehenden Schritte, wobei die Membranfiltration von Schritt d bei einem pH-Wert im Bereich von 0,1 bis 4,0 ausgeführt wird, wie zum Beispiel einem pH-Wert im Bereich von 1,3 bis 3,8, wie zum Beispiel einem pH-Wert im Bereich von 0,1 bis 3,0, wie zum Beispiel einem pH-Wert in Bereich von 0,1 bis 2,5, wie zum Beispiel einem pH-Wert im Bereich von 0,1 bis 2,0.

## Revendications

1. Procédé pour la séparation d'une ou plusieurs protéines de pomme de terre d'un groupe d'impuretés dans une solution aqueuse, comprenant ;
a. fournir une solution aqueuse comprenant l'une ou plusieurs protéines de pomme de terre et un groupe d'impuretés
b. facultativement soumettre la solution aqueuse à un prétraitement pour clarifier et éliminer des matières non solubles suspendues
c. précipiter l'une ou plusieurs protéines de pomme de terre pour créer une suspension de protéines de pomme de terre précipitées dans la solution contenant des impuretés
d. soumettre la suspension à un procédé de filtration membranaire où au moins un élément du groupe d'impuretés passe la membrane comme un perméat et l'une ou plusieurs protéines de pomme de terre précipitées sont concentrées dans le rétentat
e. facultativement diafiltrer le rétentat avec un ou plusieurs solvants pour éliminer davantage d'impuretés dans le perméat
f. facultativement resolubiliser le précipité d'une ou plusieurs protéines de pomme de terre
g. facultativement séparer le groupe d'impuretés dans ledit perméat en au moins deux fractions individuelles.

2. Procédé selon la revendication 1, comprenant ;
a-1. fournir une solution aqueuse comprenant de la patatine, des inhibiteurs de protéase, des phénols et des glycoalcaloïdes,
b-1. facultativement soumettre la solution aqueuse à un prétraitement pour clarifier et éliminer des matières non solubles suspendues,
c-1. précipiter la patatine pour créer une suspension de patatine précipitée dans la solution contenant des inhibiteurs de protéase et des glycoalcaloïdes,
d-1. soumettre la suspension à un procédé de filtration membranaire où les inhibiteurs de protéase solubles, les phénols et les glycoalcaloïdes passent la membrane comme un perméat et la patatine précipitée est concentrée dans le rétentat,
e-1. diafiltrer le rétentat avec un ou plusieurs solvants pour éliminer davantage d'inhibiteurs de protéase, de phénols et de glycoalcaloïdes dans le perméat,
f-1. facultativement resolubiliser la patatine précipitée,
g-1. séparer les inhibiteurs de protéase et les glycoalcaloïdes en deux fractions individuelles.

3. Procédé selon la revendication 1 comprenant ;
a-2 fournir une solution aqueuse comprenant de la patatine, des inhibiteurs de protéase, des phénols et des glycoalcaloïdes,
b-2 facultativement soumettre la solution aqueuse à un prétraitement pour clarifier et éliminer des matières non solubles suspendues,
c-2 précipiter la patatine pour créer une suspension de patatine précipitée dans la solution contenant des inhibiteurs de protéase et des glycoalcaloïdes,
d-2 soumettre la suspension à un procédé de filtration membranaire où des phénols et des glycoalcaloïdes passent la membrane comme un perméat et la patatine précipitée et les inhibiteurs de protéase solubles sont concentrés dans le rétentat,
e-2 diafiltrer le rétentat avec un ou plusieurs solvants pour éliminer davantage de phénols et de glycoalcaloïdes dans le perméat,
f-2 facultativement resolubiliser la patatine précipitée,
g-2 facultativement séparer la patatine et les inhibiteurs de protéase en deux fractions individuelles.

4. Procédé selon l'une quelconque des revendications 1 à 3 où ladite étape de prétraitement b) est obligatoire.

5. Procédé selon l'une quelconque des revendications 1 à 4 où ladite étape de resolubilisation f) est obligatoire.

6. Procédé selon l'une quelconque des revendications 1 à 5 où ladite étape de séparation g) est obligatoire.

7. Procédé selon l'une quelconque des revendications précédentes où l'étape de prétraitement b) comprend l'ajout d'une ou plusieurs substances pour floculer les matières non solubles suspendues, où de préférence l'étape de prétraitement b) comprend l'ajout d'ions de calcium ou d'ions de magnésium à la solution, plus préférablement où l'étape de prétraitement b) comprend l'ajout d'ions de phosphate et d'ions de calcium ou d'ions de magnésium à la solution.

8. Procédé selon l'une quelconque des revendications précédentes où l'étape de prétraitement b) est effectuée à un pH dans la gamme de pH 4,0 à pH 9,0, tel que dans la gamme de pH 4,5 à pH 6,5 tel que dans la gamme de pH 4,7 pour pH 6,0, tel que dans la gamme de pH 4,7 à pH 5,8, tel que dans la gamme de pH 4,8 à pH 5,7.

9. Procédé selon l'une quelconque des revendications précédentes, où la précipitation à l'étape c) est effectuée à un pH dans la gamme de 0,1 à 5,0, tel qu'un pH dans la gamme de 0,1 à 4,5, tel qu'un pH dans la gamme de de 0,5 à 4,5, tel qu'un pH dans la gamme de 0,8 à 4,5, tel qu'un pH dans la gamme de 0,9 à 4,5, tel qu'un pH dans la gamme de 1,0 à 4,5, tel qu'un pH dans la gamme de 1,3 à 4,5, tel qu'un pH dans la gamme de 1,3 à 3,8, tel qu'un pH dans la gamme de 0,1 à 4,0, tel qu'un pH dans la gamme de 0,5 à 4,0, tel qu'un pH dans la gamme de 1,0 à 4,0 , tel qu'un pH dans la gamme de 0,5 à 3,5, tel qu'un pH dans la gamme de 1,0 à 3,3.

10. Procédé selon l'une quelconque des revendications précédentes où la précipitation à l'étape c) comprend l'ajout d'un polymère anionique, où de préférence le polymère anionique est un polymère comestible choisi parmi le groupe d'alginate, du carraghénane, de la carboxyméthylcellulose, du carboxyméthylamidon, du carboxyméthyle et du dextrane.

11. Procédé selon l'une quelconque des revendications précédentes où le procédé de filtration membranaire de la phase d) est un procédé de filtration de flux tangentiel où la membrane est une membrane de microfiltration avec une taille de pore dans la gamme de 0,05 micron à 5 micron, telle que dans la gamme de 0,05 micron à 3 micron, telle que dans la gamme de 0,05 micron à 2 micron, telle que dans la gamme de 0,1 à 1,8 micron, telle que dans la gamme de 0,2 micron à 2 micron, telle que dans la gamme de 0,5 à 1,7 micron.

12. Procédé selon l'une quelconque des revendications 1 à 10, où le procédé de filtration membranaire de l'étape d) est un procédé de filtration à flux tangentiel effectué avec une membrane d'ultrafiltration ayant une taille de pores dans la gamme de 1 000 D à 1 000 000 D, telle que dans la gamme de 5 000 D à 200 000 D, telle que dans la gamme de 7 000 D à 150 000 D, telle que dans la gamme de 8 000 D à 100 000 D, telle que dans la gamme de 5 000 D à 30 000 D, telle que dans la gamme de 30 000 D à 1 000 000 D, telle que dans la gamme de 50 000 D à 1 000 000 D, telle que dans la gamme de 100 000 D à 1 000 000 D, telle que dans la gamme de 250 000 D à 1 000 000 D.

13. Procédé selon l'une quelconque des revendications précédentes, où le procédé membranaire de l'étape d) est un procédé de filtration à flux tangentiel effectué dans un système de membrane à fibres creuses, tubulaires ou à disque rotatif ou où le procédé membranaire de l'étape d) est un procédé de filtration à flux tangentiel effectué dans un système de membrane céramique, ou où le procédé membranaire de l'étape d) est un procédé de filtration à flux tangentiel effectué dans un système de membrane polymère enroulée en spirale.

14. Procédé selon l'une quelconque des revendications précédentes, où la diafiltration de l'étape e) comprend une diafiltration à un pH dans la gamme de 0,1 à 4,0, tel qu'un pH dans la gamme de 1,3 à 3,8, tel qu'un pH dans la gamme de 0,1 à 3,0, tel qu'un pH dans la gamme de 0,1 à 2,5, tel qu'un pH dans la gamme de 0,1 à 2,0

15. Procédé selon l'une quelconque des étapes précédentes, où la filtration membranaire de l'étape d est effectuée à un pH dans la gamme de 0,1 à 4,0, tel qu'un pH dans la gamme de 1,3 à 3,8, tel qu'un pH dans la gamme de 0,1 à 3,0, tel qu'un pH dans la gamme de 0,1 à 2,5, tel qu'un pH dans la gamme de 0,1 à 2,0.
